(19) Europäisches Patentamt
European Patent Office
Office européen des brevets

(11) EP 1 826 245 A1

(12) **EUROPEAN PATENT APPLICATION**
published in accordance with Art. 158(3) EPC

(43) Date of publication:
**29.08.2007 Bulletin 2007/35**

(21) Application number: **05814269.6**

(22) Date of filing: **09.12.2005**

(51) Int Cl.:
*C09B 23/00* (2006.01) *C07D 209/10* (2006.01)
*C09B 67/48* (2006.01) *C09K 3/00* (2006.01)

(86) International application number:
**PCT/JP2005/022653**

(87) International publication number:
**WO 2006/064731 (22.06.2006 Gazette 2006/25)**

(84) Designated Contracting States:
**BE CH DE FR GB IT LI**

(30) Priority: **15.12.2004 JP 2004362825**

(71) Applicant: **Yamamoto Chemicals, Inc.**
**Yao-shi,**
**Osaka 581-0034 (JP)**

(72) Inventors:
- **FUJITA, Shigeo,**
  **c/o Yamamoto Chemicals, Inc.**
  **Yao-shi,**
  **Osaka 581-0034 (JP)**
- **CHICHIISHI, Keiki,**
  **c/o Yamamoto Chemicals, Inc.**
  **Yao-shi,**
  **Osaka 581-0034 (JP)**
- **WADA, Sayuri,**
  **c/o Yamamoto Chemicals, Inc.**
  **Yao-shi,**
  **Osaka 581-0034 (JP)**
- **EDA, Tsunehito,**
  **c/o Yamamoto Chemicals, Inc.**
  **Yao-shi,**
  **Osaka 581-0034 (JP)**
- **TERAO, Hiroshi,**
  **c/o Yamamoto Chemicals, Inc.**
  **Yao-shi,**
  **Osaka 581-0034 (JP)**

(74) Representative: **Perry, Robert Edward**
**Gill Jennings & Every LLP**
**Broadgate House**
**7 Eldon Street**
**London EC2M 7LH (GB)**

# (54) NONSOLVATED CRYSTALS OF POLYMETHINE COMPOUND, PROCESS FOR PRODUCTION THEREOF AND USE OF THE CRYSTALS

(57) The object is to provide a novel nonsolvate-form crystal of polymethine compound which has good stability in solution, shows a high gram extinction coefficient, is excellent in storage stability, is easy to handle and is highly sensitive to general-purpose semiconductor lasers.

Thus are provided a nonsolvate-form crystal of a polymethine compound of the formula (I) and a process for producing the nonsolvate-form crystal of polymethine compound of formula (I) which comprises reacting a polymethine ether compound of the formula (II) given below with p-toluenesulfonic acid.

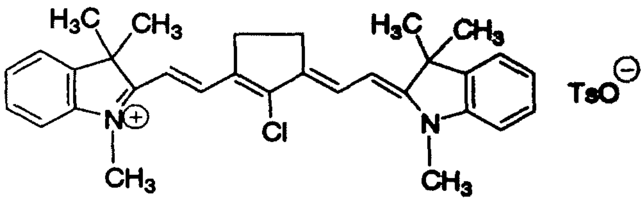 (I)

(In the above formula, TsO represents the p-toluenesulfonic acid residue.)



Printed by Jouve, 75001 PARIS (FR)

(Cont. next page)

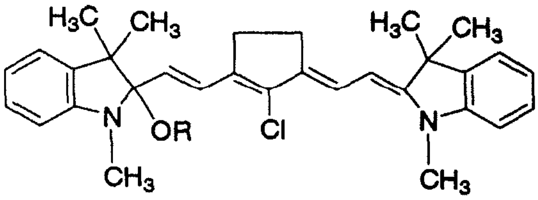

(II)

(In the above formula, R represents an alkyl group, an alkoxyalkyl group or an optionally substituted aryl group.)

## Description

Technical Field

The present invention relates to a novel nonsolvate-form crystal of a polymethine compound, a process for producing the same, and a near-infrared absorbing material using the nonsolvate-form crystal.

Background Art

In recent years, polymethine compounds have come into wide use, among others, as materials for optical recording media and near-infrared absorbing filters, or as light-to-heat converting agents in materials for plate making utilizing laser beams. In the field of materials for plate making utilizing laser beams, in particular, the demand for compounds which are highly sensitive to laser beams emitted by general-purpose semiconductor lasers, for example in the laser wavelength range of 780 nm to 840 nm, and are fairly soluble in general-purpose solvents, for example alcohols such as methanol and ethanol, has recently been increasing. Further, it is also important that such compounds be stable and easy to handle and free of impurities possibly producing adverse effects in various fields of application. However, any polymethine compound capable of satisfying such requirements is not known.

Since certain polymethine compounds were disclosed in Zh. Org. Khim. (1978), 14 (10), various investigations have been made concerning compounds similar in structural formula itself to the polymethine compounds of the present invention. According to the known methods of synthesizing such compounds, an indolenium compound represented by the formula (III)

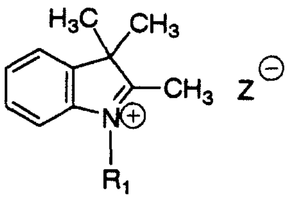

(III)

wherein $R_1$ represents an alkyl group, which may optionally be substituted, and Z represents an acidic residue, or an indoline compound represented by the general formula (IV)

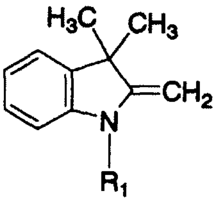

(IV)

wherein $R_1$ represent an alkyl group, which may optionally be substituted, for instance, is condensed with a diformyl compound represented by the formula (V) or a dianil compound represented by the formula (VI)

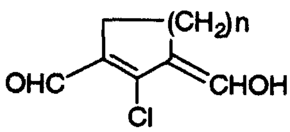

(V)

n=1 or 2

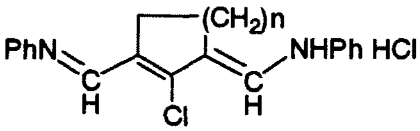

(VI)

n=1 or 2

in a dehydrating organic acid in the presence of an organic amine or a fatty acid salt (cf. e.g. Patent Document 1: Japanese Kokai (Laid-open) Publication 2001-64255; Patent Document 2: Japanese Kokai Publication 2002-52855; Patent Document 3: Japanese Kokai Publication H10-195319, pages 8-10; Patent Document 4: Japanese Patent Specification No. 3045404, Example 1; Patent Document 5: German Laid-open Patent Specification DE 3721850; Patent Document 6: Japanese Kokai Publication S62-36469; Non-Patent Document 1: J. Org. Chem. 1995, 60, 2394).

Among them, the method of synthesizing polymethine compounds by reacting an indolenium compound of formula (III) with a dianil compound of formula (VI) is the commonest. In that case, however, the compounds are limited in type or kind from the viewpoint of reaction yield of the product polymethine compound and ease of operation in isolation and purification, among others. Such methods that use a compound of general formula (VI) or a compound of general formula (V), when n is 2, are generally used in producing polymethine compounds in which Z is the perchloric acid residue, tetrafluoroboric acid residue or p-toluenesulfonic acid residue, as disclosed in Patent Document 1. However, there are few examples of synthesis for the cases where n in the formula is 1; in particular, nothing is known about the production of polymethine compounds in which $Z^-$ is the p-toluenesulfonic acid residue.

As for the method comprising reacting an indoline compound of formula (IV) with a diformyl compound of formula (V), synthesis examples in which the counter ion to a polymethine compound is the p-toluenesulfonic acid residue are disclosed in Synthesis Example 1 and Synthesis Example 2 in Patent Document 2. However, the compounds obtained by the method of synthesis described in this document differ in basic structural formula from the polymethine compound of the present invention and no physical characteristic values are described in that document. When a compound having the same structure as that of the polymethine compound of the present invention was produced by the production method disclosed there, the yield was low and the compound obtained was low in purity and in a hydrated form. Further, the diformyl compound of formula (V) as used therein is poor in storage stability and hazardous (positive in mutage-necity testing) and, therefore, caution is necessary in handling the same and the use thereof as a raw material for commercial scale production is undesirable.

As for the method comprising reacting an indoline compound of formula (IV) with a dianil compound of formula (VI), a compound differing in basic structural formula from that of the polymethine compound of the present invention is disclosed in Example 3 of Patent Document 6. When a compound structurally identical to the polymethine compound of the present invention was produced by the production method disclosed in that document, the substance obtained was a methanol adduct and was a low-purity compound.

In Patent Document 7 (Japanese Kokai Publication 2000-35669), an example is described of the use of a compound identical in basic structural formula to the polymethine compound of the present invention as an infrared absorbing material in a negative type image recording material (for use in the field of plate making utilizing laser beams). However, there is no description about the process for producing the polymethine compound itself or about the physical characteristics thereof; there is no description about the stability or sensitivity thereof.

All the compounds disclosed in such documents as cited above are the products produced based on the prior art methods mentioned above and differ in physical characteristics from the polymethine compound obtainable by the process for producing the polymethine compound according to the invention and showing no solvate formation. The known compound identical in structural formula to the compound of the invention occurs as a solvate due to the process for production thereof and is a low-purity product and, therefore, the use thereof is greatly restricted. When the solvate-form compound structurally identical to the compound of the present invention is used as a light-to-heat converting agent in plate making by the CTP (computer-to-plate) technique, practical difficulties are encountered, namely the solution stability is poor, and the light-to-heat conversion efficiency widely fluctuates due to the fact that the purity is not constant.

The term "solvate" as used herein is a generic one including the hydrate. Meanwhile, no report can be found about the fact that in the case of polymethine compounds, there are great differences in stability in solution and in sensitivity between the solvated form and non-solvated form in spite of the same basic structure of the compound.

Disclosure of Invention

*Problems Which the Invention is to Solve*

It is an object of the present invention to provide a novel nonsolvate-form crystal of a polymethine compound, which is very stable in solution, shows a high gram extinction coefficient, is highly pure, stable and easy to handle and is highly sensitive to beams emitted by general-purpose semiconductor lasers.

*Means for Solving the Problems*

The present inventors made various investigations in an attempt to solve the problems discussed above and, as a result, found that a novel nonsolvate-form crystal of a polymethine compound having a specific structure shows good stability in solution and a high gram extinction coefficient, is highly sensitive to laser beams around 780 nm to 840 nm and highly pure and stable and can be used as a near-infrared absorbing material readily processable in various fields of application. Based of such and other findings, they have now completed the present invention.

Thus, the invention in the instant application consists in the following:

(1) A nonsolvate-form crystal of a polymethine compound represented by the formula (I):

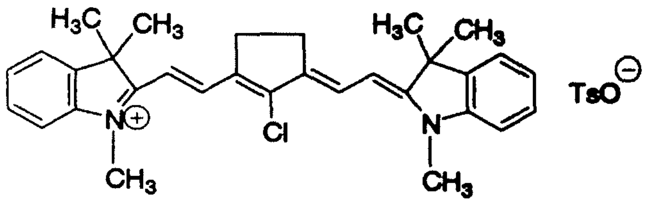

(I)

wherein TsO represents the p-toluenesulfonic acid residue.

(2) The nonsolvate-form crystal of polymethine compound as defined above under (1) which shows a thermogravimetric (TG) weight loss not exceeding 3% at up to 150°C in a TG-TDA (thermogravimetry-differential thermal analysis) chart thereof.

(3) A process for producing the nonsolvate-form crystal of polymethine compound as defined above under (1) or (2) which comprises reacting a polymethine ether compound represented by the formula (II) with p-toluenesulfonic acid.

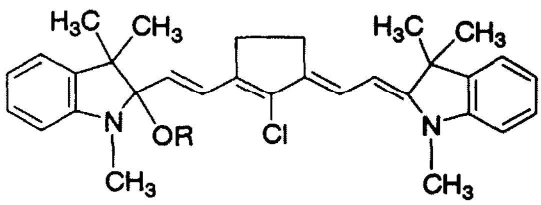

(II)

In the above formula, R represents an alkyl or alkoxyalkyl group or an aryl group which may optionally be substituted.

(4) The $\alpha$ crystal modification of the nonsolvate-form crystal of polymethine compound as defined above under (1) or (2) which is characterized by a powder X-ray diffraction pattern thereof showing characteristic peaks at diffraction angles ($2\theta \pm 0.2°$) of 11.7°, 16.3°, 17.0°, 24.8° and 27.5° in powder X-ray diffractometry using the Cu-K$\alpha$ rays.

(5) A process for producing the $\alpha$ crystal modification of the nonsolvate-form crystal of polymethine compound as defined above under (4) which comprises using a polymethine ether compound represented by the formula (II) and p-toluenesulfonic acid as reactants and obtaining the crystal in the presence of a mixed solvent composed of a ketone solvent and an ester solvent.

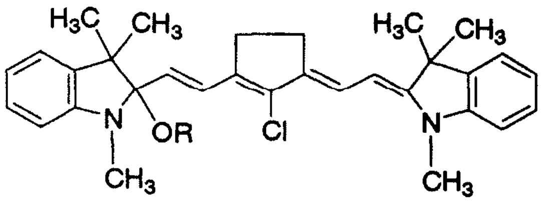

(II)

In the above formula, R represents an alkyl or alkoxyalkyl group or an aryl group which may optionally be substituted.

(6) The β crystal modification of the nonsolvate-form crystal of polymethine compound as defined above under (1) or (2) which is characterized by a powder X-ray diffraction pattern thereof showing characteristic peaks at diffraction angles (2θ ± 0.2°) of 15.5°, 19.0°, 22.3° and 22.9° in powder X-ray diffractometry using the Cu-Kα rays.

(7) A process for producing the β crystal modification of the nonsolvate-form crystal of polymethine compound as defined above under (6) which comprises subjecting the α crystal modification defined above under (4) to dispersion treatment in warm water at 30°C or above.

(8) The γ crystal modification of the nonsolvate-form crystal of polymethine compound as defined above under (1) or (2) which is characterized by a powder X-ray diffraction pattern thereof showing characteristic peaks at diffraction angles (2θ ± 0.2°) of 11.6°, 11.9° and 24.5° in powder X-ray diffractometry using the Cu-Kα rays.

(9) A process for producing the γ crystal modification defined above under (8) which comprises using a polymethine ether compound represented by the formula (II) and p-toluenesulfonic acid as reactants and causing the crystal to precipitate out from a mixed solvent composed of an alcohol solvent and water.

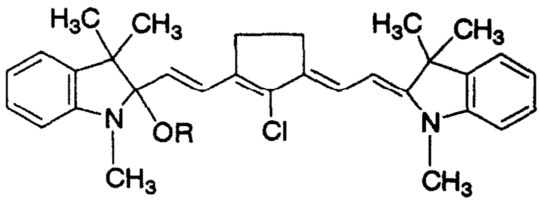

(II)

In the above formula, R represents an alkyl or alkoxyalkyl group or an aryl group which may optionally be substituted.

(10) The δ crystal modification of the nonsolvate-form crystal of polymethine compound as defined above under (1) or (2) which is characterized by a powder X-ray diffraction pattern thereof showing characteristic peaks at diffraction angles (2θ ± 0.2°) of 10.8°, 14.8°, 16.9°, 21.4° and 23.7° in powder X-ray diffractometry using the Cu-Kα rays.

(11) A process for producing the δ crystal modification defined above under (10) which comprises dissolving at least one of the α, β and γ crystal modifications defined above under (4), (6) and (8), respectively, in a mixed solvent composed of a ketone solvent and an alcohol solvent and causing the crystal to precipitate out therefrom by addition of an ester solvent.

(12) A process for producing the δ crystal modification defined above under (10) which comprises using a polymethine ether compound represented by the formula (II) and p-toluenesulfonic acid as reactants, carrying out the reaction using a mixed solvent composed of a ketone solvent and an alcohol solvent and then adding an ester solvent to the reaction mixture for causing the crystal to precipitate out.

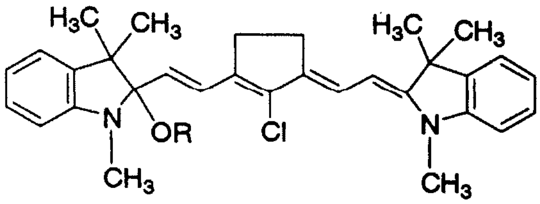

(II)

In the above formula, R represents an alkyl or alkoxyalkyl group or an aryl group which may optionally be substituted.

(13) A near-infrared absorbing material characterized by containing the nonsolvate-form crystal of polymethine compound as defined above under (1), (2), (4), (6), (8) or (10).

Detailed Description of the Invention

In the following, the invention is described in detail.

The known compound represented by the chemical structural formula (I) or the compound of formula (I) as obtained by the known production methods is a substance solvated with water or an organic solvent (e.g. methanol, ethanol) and, in most cases, is low in purity. On the contrary, the nonsolvate-form crystal of polymethine compound of formula (I) according to the invention is in a quite novel crystal form not solvated with water or any organic solvent.

The nonsolvate-form crystal of polymethine compound of formula (I) according to the invention gives a TG-DTA (thermogravimetry-differential thermal analysis) chart showing a TG weight loss not exceeding 3%, preferably not exceeding 2%, at 150°C. The solvated and/or low-purity products often show TG weight losses exceeding 3% at 150°C.

Further, it was found that the nonsolvate-form crystal of polymethine compound of formula (I) according to the invention can occur in any of four crystal modification forms depending on the manner of recovery of the crystal. In the instant application, these four crystal modifications are referred to as α crystal modification, β crystal modification, γ crystal modification and δ crystal modification, respectively.

These crystal modifications each shows respective particular characteristic peaks in a diffraction pattern in powder X-ray diffractometry using the Cu-Kα rays. The solvate-form and/or low-purity products show quite different powder X-ray diffraction patterns.

These crystal modifications each shows a respective definite melting point (decomposition temperature) of not lower than 190°C whereas the solvate-form and/or low-purity products often show no definite melting point (decomposition temperature) or show a melting point (decomposition temperature) lower than 190°C.

It has been revealed that the nonsolvate-form crystal of polymethine compound of the invention can be produced only via (by using as the raw material) a polymethine ether compound represented by the formula (II).

Surprisingly, the nonsolvate-form crystal of polymethine compound of formula (I) according to the invention is higher in solution stability, as compared with the solvate-form compounds known in the art, in those solvents which are used in the field of plate making utilizing laser beams and in the field of laser heat sensitive recording materials, among others, for example alcohol solvents such as methanol and ethanol and ketone solvents such as acetone and methyl ethyl ketone, hence is very suited for use in these fields of application. In addition, it shows a high extinction coefficient in the region of 780-840 nm and therefore can be properly used in a number of recoding material fields where laser beams (emission wavelength range: 780-840 nm) emitted by general-purpose semiconductor lasers are utilized; further, it is very useful in the field of such recording materials as laser thermal transfer recording materials and laser heat sensitive recording materials, and in the field of plate making materials.

[Process for producing the nonsolvate-form crystal of polymethine compound]

The nonsolvate-form crystal of polymethine compound of formula (I) according to the invention can be produced by reacting a polymethine ether compound represented by the general formula (II) (e.g. R = $CH_3$) with p-toluenesulfonic acid.

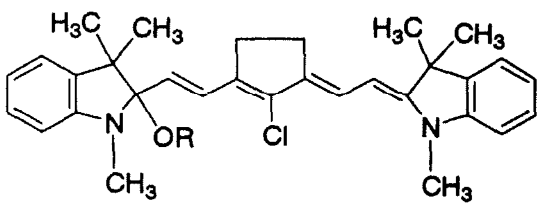

(II)

In the above formula, R represents an alkyl or alkoxyalkyl group or an aryl group which may optionally be substituted.

When R is an alkyl group, it is preferably a straight or branched alkyl group containing 1-8 carbon atoms, particularly preferably a straight or branched alkyl group containing 1-4 carbon atoms. As examples, there may be mentioned methyl, ethyl, n-propyl, isopropyl, n-butyl, isobutyl, sec-butyl, tert-butyl, n-pentyl, isopentyl, neopentyl, n-hexyl, isohexyl, sec-hexyl, 2-ethylbutyl, n-heptyl, isoheptyl, sec-heptyl, n-octyl and 2-ethylhexyl.

When R is an alkoxyalkyl group, it is preferably one containing 2-8 carbon atoms in total, particularly preferably one containing 2-4 carbon atoms in total. As example, there may be mentioned methoxymethyl, 2-methoxyethyl, 3-methoxypropyl, 2-ethoxymetyl, 2-ethoxyethyl, 2-propoxyethyl and 2-butoxyethyl.

When R is an aryl group which may optionally be substituted, it may be an optionally substituted phenyl group or an optionally substituted naphthyl group but preferably is an optionally substituted phenyl group. Each substituent may be an alkyl, amino, nitro, alkoxy or hydroxy group or a halogen atom, and preferably is an alkyl group containing

1-4 carbon atoms or an alkoxy group containing 1-4 carbon atoms.

As examples of R when it is an alkyl-substituted phenyl, there may be mentioned 2-methylphenyl, 3-methylphenyl, 4-methylphenyl, 2,3-dimethylphenyl, 2,4-dimethylphenyl, 3,4-dimethylphenyl, 2,5-dimethylphenyl, 2,6-dimethylphenyl, 2-ethylphenyl, 3-ethylphenyl, 4-ethylphenyl, 2,3-diethylphenyl, 2,4-diethylphenyl, 3,4-diethylphenyl, 2,5-diethylpehnyl and 2,6-diethylphenyl.

As examples of R when it is an alkoxy-substituted phenyl, there may be mentioned 2-methoxyphenyl, 3-methoxyphenyl, 4-methoxyphenyl, 2,3-dimethoxyphenyl, 2,4-dimethoxyphenyl, 3,4-dimethoxyphenyl, 2,5-dimethoxyphenyl and 2,6-dimethoxyphenyl.

As for the amounts of the compound of formula (II) and p-toluenesulfonic acid to be used, the latter is used generally in an amount of about 0.5 to 3 moles, preferably about 1 to 1.5 moles, per mole of the former.

The solvent and reaction and after-treatment conditions to be employed vary according to the crystal modification to be produced.

After the reaction, the desired product can be readily isolated by filtration and washing. The product can be readily purified by conventional purification means, for example recrystallization.

The polymethine ether compound (II) mentioned above can be prepared, for example, by reacting a polymethine compound represented by the formula (VII) given below with an alkali metal alkoxide or alkali metal aryloxide represented by the formula (VIII) given below in an organic solvent.

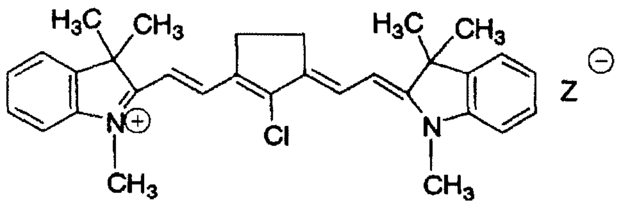

(VII)

(In the formula, $Z^-$ represents an acidic residue.)

MOR (VIII)

(In the formula, M represents an alkali metal and R is as defined above.)

In the above formula (VII), $Z^-$ represents an acidic residue, for example $F^-$, $Cl^-$ $Br^-$, $I^-$, $BrO_4^-$, $ClO_4^-$, $BF_4^-$, $PF_6^-$, $SbF_6^-$, $CF_3CO_2^-$, $CH_3CO_2^-$, $CF_3SO_3^-$, $CH_3SO_3^-$, benzenecarbonato, benzenesulfonato, p-toluenesulfonato (hereinafter referred to as $TsO^-$ for short), naphthalenecarbonato, naphthalenedicarbonato, naphthalenesulfonato, naphthalenedisulfonato or the like. In particular, $Cl^-$ $Br^-$, $I^-$, $ClO_4^-$ , $BF_4^-$, $PF_6^-$, $SbF_6^-$, $CF_3CO_2^-$, $CF_3SO_3^-$, $CH_3SO_3^-$, benzenecarbonato, benzenesulfonato and $TsO^-$ are preferred, and $ClO_4^-$, $BF_4^-$ and $TsO^-$ are more preferred. The polymethine compound of formula (VII) to be used here may be in any solvated crystal form known in the art.

In the above reaction, an alkali metal, for example sodium or potassium, is used as M.

As the organic solvent, there may be mentioned alcohols such as methanol, ethanol, n-propanol, isopropanol and n-butanol, ethers such as tetrahydrofuran and dioxane, esters such as methyl acetate, ethyl acetate and butyl acetate, aromatic hydrocarbons such as benzene, toluene and xylene, halogenated hydrocarbons such as dichloromethane, trichloromethane, dichloroethane and trichloroethane, and aprotic polar solvents such as dimethylformamide, dimethylacetamide and dimethyl sulfoxide.

As for the proportion between the compound represented by the general formula (VII) and the compound represented by the general formula (VIII), the latter is used generally in an amount of about 1-30 moles, preferably about 2-10 moles, per mole of the former.

The organic solvent is used generally in an amount of about 2-30 liters, preferably about 5-20 liters, per mole of the compound represented by the general formula (VII).

Generally, the above reaction proceeds smoothly at a temperature of about 0-100°C, preferably at 10-70°C, and generally will be complete in about several minutes to about 10 hours.

After reaction, the desired product can be isolated with ease by filtration and washing. It can be purified with ease by any of conventional means of purification, for example recrystallization or column separation.

The compound represented by the general formula (VII) can be synthesized by the method described in Japanese Kokai Publication 2000-226528, for instance.

[α crystal modification]

The α crystal modification of the nonsolvate-form crystal of polymethine compound according to the invention in the instant application shows characteristic peaks at diffraction angles (2θ ± 0.2°) of 11.7°, 16.3°, 17.0°, 24.8° and 27.5°, preferably at 11.7°, 12.1°, 16.3°, 17.0°, 18.3°, 24.8° and 27.5°, in powder X-ray diffractometry using the Cu-Kα rays.
The α crystal modification can be produced in the following manner.
A polymethine ether compound represented by the general formula (II) and p-toluenesulfonic acid are used as reactants, and the crystal is obtained in the presence of a mixed solvent composed of a ketone solvent and an ester solvent.
The ketone solvent includes, among others, acetone, methyl ethyl ketone, methyl propyl ketone and methyl butyl ketone. Among them, acetone and methyl ethyl ketone are preferred, and acetone is particularly preferred.
The ester solvent includes, among others, methyl acetate, ethyl acetate and butyl acetate and, among them, ethyl acetate is preferred.
The ketone solvent is used in an amount of 2-30 times, preferably 5-15 times, the amount of the polymethine ether compound represented by the general formula (II) on the volume/weight ratio basis.
The ester solvent is used in an amount of 2-10 times, preferably 3-7 times, the amount of the polymethine ether compound represented by the general formula (II) on the volume/weight ratio basis.
As for the above-mentioned reaction, the polymethine ether compound represented by the general formula (II) and p-toluenesulfonic acid are first subjected to reaction in a ketone solvent at 0-60°C, preferably at 10-50°C, for 10 minutes to 10 hours, preferably for 30 minutes to 2 hours, and an ester solvent is then added, and the mixture is maintained at 0-60°C, preferably at 40-60°C, for 10 minutes to 10 hours, preferably for 30 minutes to 2 hours for completion of the reaction.
During the reaction between the reactants mentioned above in the ketone solvent, crystals gradually precipitate out and, during heating following addition of the ester solvent, a large amount of crystals precipitate out in the form of α crystal modification. After completion of the reaction, the reaction mixture is cooled to 20°C or below, the precipitate crystals are collected by filtration, washed with the ester solvent and dried to give crystals in the form of α crystal modification.

[β crystal modification]

The β crystal modification of the nonsolvate-form crystal of polymethine compound according to the invention in the instant application shows characteristic peaks at diffraction angles (2θ ± 0.2°) of 15.5°, 19.0°, 22.3° and 22.9°, preferably at 15.5°, 16.1°, 19.0°, 22.3°, 22.9° and 23.8°, in powder X-ray diffractometry using the Cu-Kα rays.
The β crystal modification can be produced by subjecting the α crystal modification to dispersion treatment in warm water at 30°C or above.
The warm water is used in an amount of 1-100 times, preferably 5-15 times, the amount of the α crystal modification on the volume/weight ratio basis.
The temperature of warm water is preferably not lower than 30°C, more preferably 40-80°C. Even when the temperature of warm water is lower than 30°C, the β crystal modification can be obtained but a long period of time is required for the crystal form conversion treatment.
The time required for dispersion treatment is 10 minutes to 5 hours, preferably 20 minutes to 2 hours.
After completion of the treatment, crystals are collected by filtration, washed with water and dried to give the β crystal modification.

[γ crystal modification]

The γ crystal modification of the nonsolvate-form crystal of polymethine compound according to the invention in the instant application shows characteristic peaks at diffraction angles (2θ ± 0.2°) of 11.6°, 11.9° and 24.5°, preferably at 5.6°, 11.6°, 11.9°, 16.8°, 24.5° and 24.9°, in powder X-ray diffractometry using the Cu-Kα rays.
The γ crystal modification can be produced in the following manner.
A polymethine ether compound represented by the general formula (II) and p-toluenesulfonic acid are used as reactants, and the crystal is obtained in the presence of a mixed solvent composed of an alcohol solvent and water.
The alcohol solvent includes, among others, methanol, ethanol, n-propanol, isopropanol and n-butanol. Among them, methanol and ethanol are preferred, and methanol is particularly preferred.
The alcohol solvent is used in an amount of 7-30 times, preferably 8-15 times, the amount of the polymethine ether compound represented by the general formula (II) on the volume/weight ratio basis.
Water is used in an amount of 7-30 times, preferably 8-15 times, the amount of the polymethine ether compound represented by the general formula (II) on the volume/weight ratio basis.
When the amounts of the alcohol solvent and water are each smaller than 7 times the amount of (11), the

crystals obtained may be found contaminated with the hydrate or alcohol solvate form in some instances.

As for the above-mentioned reaction, the polymethine ether compound represented by the general formula (II) and p-toluenesulfonic acid are first subjected to reaction in an alcohol solvent at 0-70°C, preferably at 20-40°C, for 10 minutes to 10 hours, preferably for 30 minutes to 2 hours. To this reaction mixture in solution form, water is added, and the mixture is maintained at 0-50°C, preferably 20-40°C, for 10 minutes to 10 hours, preferably for 30 minutes to 2 hours, whereupon the γ crystal modification precipitates out.

When the reaction temperature after addition of water is higher than 50°C, the crystals obtained may be found contaminated with the hydrated or alcohol-solvated form in certain cases.

After completion of the reaction, the reaction mixture is cooled to 20°C or below, the precipitate crystals are collected by filtration, washed with water and dried to give crystals in the form of γ crystal modification.

[δ crystal modification]

The δ crystal modification of the nonsolvate-form crystal of polymethine compound according to the invention in the instant application shows characteristic peaks at diffraction angles (2θ ± 0.2°) of 10.8°, 14.8°, 16.9°, 21.4° and 23.7°, preferably at 10.8°, 14.8°, 16.9°, 21.4°, 22.1° and 23.7°, in powder X-ray diffractometry using the Cu-Kα rays.

The δ crystal modification can be produced in the following manner.

A polymethine ether compound represented by the general formula (II) and p-toluenesulfonic acid are used as reactants, and the reaction is carried out using a mixed solvent composed of a ketone solvent and alcohol solvent and, thereafter, an ester solvent is added to obtain the crystal.

The ketone solvent includes, among others, acetone, methyl ethyl ketone, methyl propyl ketone and methyl butyl ketone. Among them, acetone and methyl ethyl ketone are preferred, and acetone is particularly preferred.

The alcohol solvent includes, among others, methanol, ethanol, n-propanol, isopropanol and n-butanol. Among them, methanol and ethanol are preferred, and methanol is particularly preferred.

The ester solvent includes, among others, methyl acetate, ethyl acetate and butyl acetate, and ethyl acetate is preferred.

The ketone solvent is used in an amount of 1-10 times, preferably 1-3 times, the amount of the polymethine ether compound represented by the general formula (II) on the volume/weight ratio basis.

The alcohol solvent is used in an amount of 0.1-5 times, preferably 0.1-1 time, the amount of the polymethine ether compound represented by the general formula (II) on the volume/weight ratio basis.

The ester solvent is used in an amount of 1-30 times, preferably 5-15 times, the amount of the polymethine ether compound represented by the general formula (II) on the volume/weight ratio basis.

As for the above-mentioned reaction, the polymethine ether compound represented by the general formula (II) and p-toluenesulfonic acid are first subjected to reaction in a mixed solvent composed of a ketone solvent and an alcohol solvent at 0-60°C, preferably at 10-50°C, for 10 minutes to 10 hours, preferably for 30 minutes to 2 hours. Then, an ester solvent is then added to the reaction mixture in solution form, and the reaction is continued at 0-60°C, preferably at 40-60°C, for 10 minutes to 10 hours, preferably for 30 minutes to 2 hours, whereupon the δ crystal modification precipitates out.

After completion of the reaction, the reaction mixture is cooled to 20°C or below, and the precipitate crystals are collected by filtration, washed with the ester solvent and dried to give the δ crystal modification.

The δ crystal modification can also be produced by dissolving at least on species selected from among the α, β and γ crystal modifications in a mixed solvent composed of a ketone solvent and an alcohol solvent and, then, adding an ester solvent to the solution.

The kinds and amounts of the ketone solvent, alcohol solvent and ester solvent are the same as described above for the above-mentioned case of production using the polymethine ether compound and p-toluenesulfonic acid as reactants.

The step of dissolving at least on species selected from among the α, β and γ crystal modifications in a mixed solvent composed of a ketone solvent and an alcohol solvent is carried out with stirring at 0-60°C, preferably at 10-50°C, for 10 minutes to 10 hours, preferably for 30 minutes to 2 hours.

After addition of an ester solvent, the whole mixture is stirred at 0-60°C, preferably at 40-60°C, for 10 minutes to 10 hours, preferably for 30 minutes to 2 hours, whereupon the δ crystal modification precipitates out.

After completion of the reaction, the reaction mixture is cooled to 20°C or below, and the precipitate crystals are collected by filtration, washed with the ester solvent and dried to give the δ crystal modification.

[Near-infrared absorbing material]

As for the near-infrared absorbing material of the present invention, the nonsolvate-form crystal of polymethine compound of formula (I) may be used singly or in combination with an appropriate binder resin, another near-infrared

absorbing substance, a color-forming component, a coloring component and/or the like, according to need.

The binder resin is not particularly restricted but includes homopolymers and copolymers of acrylic monomers such as acrylic acid, methacrylic acid, acrylic esters, methacrylic esters, etc.; cellulosic polymers such as methylcellulose, ethylcellulose, cellulose acetate, etc.; vinyl polymers and vinyl compound copolymers such as polystyrene, vinyl chloride-vinyl acetate copolymers, polyvinylpyrrolidone, polyvinyl butyral, polyvinyl alcohol, etc.; condensation polymers such as polyesters and polyamides; rubber type thermoplastic polymers such as butadiene-styrene copolymers; and polymers produced by the polymerization and crosslinking of photopolymerizable compounds such as epoxy compounds, among others.

The near-infrared absorbing substance to be used in the near-infrared absorbing material may comprise not only the nonsolvate-form crystal of polymethine compound of general formula (I) but also any of various known near-infrared absorbing substances unless the latter defeats the object of the present invention.

The near-infrared absorbing substances which can be used concomitantly include not only such common pigments as carbon black and aniline black, but also the various pigment type and dye type colors described in Near-Infrared Absorbing Colors (pp. 45-51) in "Kagaku Kogyo (Chemical Industry)", May, 1986 issue and "Development and Market Trend of Functional Colors in the Nineties", CMC (1990), Chapter 2-2.3., such as polymethine colors (cyanine colors), phthalocyanine colors, dithiol metal complex colors, naphthoquinone and anthraquinone colors, triphenylmethane (analogous) colors, aminium and diimmo-nium colors, etc., as well as azo colors, indoaniline metal complex colors, intermolecular CT colors and so forth.

In cases where the near-infrared absorbing material of the present invention is used as a light-to-heat converting agent in materials for plate making utilizing laser beams, original plates for plate making can be produced by applying a solution of the near-infrared absorbing material in an organic solvent to supports, for example paper sheets, plastic (e.g. polyethylene, polypropylene, polystyrene)-laminated paper sheets, plates or sheets of a metal such as aluminum (including an aluminum alloy), zinc or copper, or plastic films made of cellulose diacetate, cellulose triacetate, cellulose butyrate, polystyrene terephthalate, polyethylene, polystyrene, polypropylene, polycarbonate, polyvinyl acetal or the like.

The solvent to be used in the solution to be applied is not particularly restricted but includes, among others, hydrocarbons, halogenated hydrocarbons, ethers, ketones, alcohols and cellosolves. Preferred are, however, ethers such as tetrahydrofuran and dioxane, ketones such as methyl ethyl ketone, methyl isobutyl ketone and cyclohexanone, alcohol solvents such as methanol, ethanol and propanol, and cellosolve solvents such as methylcellosolve and ethylcellosolve. Particularly preferred are ketones such as methyl ethyl ketone, methyl isobutyl ketone and cyclohexanone, and alcohol solvents such as methanol, ethanol and propanol.

In using the near-infrared absorbing material of the invention in a recording material such as a laser thermal transfer recording material or a laser thermal recording material, the near-infrared absorbing material may be used as formulated with a color-forming component or a coloring component, or a discrete layer containing a color-forming component or a coloring component may be provided. As the color-forming component or coloring component, sublimable dyes or pigments, electron-donating dye precursor-electron-accepting compound systems, and the systems heretofore explored in which images are formed by heat-induced physicochemical changes in polymerizable polymers or the like can be employed.

For example, the coloring component of a laser thermal transfer recoding material is not particularly restricted but, as pigment type components, there can be mentioned inorganic pigments such as titanium dioxide, carbon black, zinc oxide, Prussian blue, cadmium sulfide, iron oxide, chromates of lead, zinc, barium and calcium, etc., and organic pigments such as azo, thioindigo, anthraquinone, anthanthrone, triphenodioxazine, phthalocyanine, quinacridone and other pigments. The dye which can be used includes acid dyes, direct dyes, disperse dyes, oil-soluble dyes and metal-containing oil-soluble dyes, among others.

The color-forming component for a laser thermal recording material is not particularly restricted but the substances which have heretofore been utilized in thermal recoding materials can be employed. As an electron-donating dye precursor, there is employed a compound having a partial skeleton in the form of a lactone, lactam, sultone, spiropyran, ester, amide, or the like, and developing color by giving off an electron or receiving a proton, for example from an acid, with said partial skeleton being opened or cleaved on contact with an electron-accepting compound. For example, there can be mentioned triphenylmethane compounds, fluoran compounds, phenothiazine compounds, indolyl-phthalide compounds, leucoauramine compounds, rhodamine-lactam compounds, triphenylmethane compounds, triazene compounds, spiropyran compounds, fluorene compounds and so forth. As the electron-accepting compound, there can be mentioned phenolic compounds, organic acids or metal salts thereof, and hydroxybenzoic esters, among others.

When the near-infrared absorbing material of the invention is used in near-infrared absorbing filters, heat ray shielding materials or films for agricultural use, the near-infrared absorbing material is admixed with a plastic resin, if necessary together with an organic solvent, and the mixture can be molded into sheets or films by various methods so far investigated in the art, for example by injection molding or casting.

The resin that can be used is not particularly restricted but includes, among others, acrylic resins, polyethylene resins, vinyl chloride resins, vinylidene chloride resins and polycarbonate resins. The solvent to be used is not particularly

restricted but includes, for example, hydrocarbons, halogenated hydrocarbons, ethers, ketones, alcohols and cellosolves. Alcohols such as methanol, ethanol and propanol and cellosolve solvents such as methylcellosolve and ethylcellosolve, in particular, are preferred.

When the near-infrared absorbing material of the present invention is used in such optical recording materials as optical cards, a solution is prepared by dissolving the near-infrared absorbing material in an organic solvent, and the solution can be applied to such substrates as glass or plastic resin substrates by any of various techniques so far investigated, for example by spin coating.

The resin that can be used as the substrate resin is not particularly restricted but includes, among others, acrylic resins, polyethylene resins, vinyl chloride resins, vinylidene chloride resins and polycarbonate resins. The solvent to be used in spin coating is not particularly restricted but includes, for example, hydrocarbons, halogenated hydrocarbons, ethers, ketones, alcohols and cellosolves. In particular, alcohol solvents such as methanol, ethanol and propanol and cellosolve solvents such as methylcellosolve and ethylcellosolve are preferred.

Examples

The following examples and comparative examples illustrate the present invention more specifically. These examples are, however, by no means limitative of the scope of the present invention.

[Example 1] Production of the α crystal modification of the nonsolvate-form crystal of polymethine compound

To 150 ml of acetone was added 15.03 g of a polymethine ether compound represented by the formula (II) (R = $CH_3$), and 6.00 g of p-toluenesulfonic acid monohydrate was added to the mixture with stirring at 25-30°C. The resulting mixture was stirred at that temperature for 1 hour and then heated to a temperature of 50-55°C, and 68 ml of ethyl acetate was added dropwise. After 1 hour of stirring at the same temperature, the mixture was cooled to 15-20°C. The resulting crystalline precipitate was collected by filtration, washed with ethyl acetate and then dried to give 16.21 g of the α crystal modification of the compound of formula (I).

This crystal showed a solubility of not lower than 15% in each of methanol and ethanol. The elemental analysis data, melting point (decomposition temperature), absorption maximum wavelength (Amax) and gram extinction coefficient (εg) of this crystal were as follows.

Elemental analysis ($C_{38}H_{41}ClN_2O_3S$): MW = 641.3

| | C | H | N |
|---|---|---|---|
| Calculated (%) | 71.17 | 6.44 | 4.37 |
| Found (%) | 71.10 | 6.45 | 4.34 |

Melting point (°C): 199-200°C (decomposition)
λmax: 808 nm (diacetone alcohol solution)
εg: 4.42 x $10^5$ ml/g·cm

A powder X-ray diffraction pattern of the crystal obtained is shown in Fig. 1.

An IR spectrum of the crystal obtained is shown in Fig. 5.

A TG-DTA (thermogravimetry-differential thermal analysis) chart of the crystal obtained is shown in Fig. 9. The TG loss in weight as found by TG-DTA (not higher than 150°C) was 0.06%. In DTA, two exothermic peak temperatures were found at 208.3°C and 211.0°C (temperature programming: 5.0°C/minute).

[Example 2] Production of the β crystal modification of the nonsolvate-form crystal of polymethine compound

The α crystal modification (10.00 g) obtained in Example 1 was added to 100 ml of water at 40-45°C, the mixture was stirred at the same temperature for 1 hour, and the precipitate was collected by filtration, washed with water and dried to give 9.58 g of the β crystal modification of the compound of formula (I).

This crystal showed a solubility of not lower than 15% in each of methanol and ethanol. The elemental analysis data, melting point (decomposition temperature), absorption maximum wavelength (Amax) and gram extinction coefficient (εg) of this crystal were as follows.

Elemental analysis ($C_{38}H_{41}ClN_2O_3S$): MW = 641.3

| | C | H | N |
|---|---|---|---|
| Calculated (%) | 71.17 | 6.44 | 4.37 |

(continued)

| Elemental analysis ($C_{38}H_{41}ClN_2O_3S$): MW = 641.3 | | | |
|---|---|---|---|
| | C | H | N |
| Found (%) | 71.01 | 6.48 | 4.33 |

Melting point (°C): 201-203°C (decomposition)
λmax: 808 nm (diacetone alcohol solution)
εg: 4.41 x $10^5$ml/g·cm

A powder X-ray diffraction pattern of the crystal obtained is shown in Fig. 2.

An IR spectrum of the crystal obtained is shown in Fig. 6.

A TG-DTA (thermogravimetry-differential thermal analysis) chart of the crystal obtained is shown in Fig. 10. The TG loss in weight as found by TG-DTA (not higher than 150°C) was 1.23%. In DTA, two exothermic peak temperatures were found at 194.3°C and 203.3°C (temperature programming: 5.0°C/minute).

[Example 3] Production of the γ crystal modification of the nonsolvate-form crystal of polymethine compound

To 165 ml of methanol was added 15.03 g of a polymethine ether compound represented by the formula (II) (R = $CH_3$), and 6.00 g of p-toluenesulfonic acid monohydrate was added to the mixture with stirring at 25-30°C. The resulting mixture was stirred at that temperature for 1 hour, and 165 ml of water was added dropwise. After 1 hour of stirring at the same temperature, the mixture was cooled to 15-20°C. The resulting crystalline precipitate was collected by filtration, washed with water and then dried to give 18.04 g of the γ crystal modification of the compound of formula (I).

This crystal showed a solubility of not lower than 15% in each of methanol and ethanol. The elemental analysis data, melting point (decomposition temperature), absorption maximum wavelength (λmax) and gram extinction coefficient (εg) of this crystal were as follows.

| Elemental analysis ($C_{38}H_{41}ClN_2O_3S$): MW = 641.3 | | | |
|---|---|---|---|
| | C | H | N |
| Calculated (%) | 71.17 | 6.44 | 4.37 |
| Found (%) | 71.05 | 6.42 | 4.35 |

Melting point (°C): 198-199°C (decomposition)
λmax: 808 nm (diacetone alcohol solution)
εg: 4.41 x $10^5$ml/g·cm

A powder X-ray diffraction pattern of the crystal obtained is shown in Fig. 3.

An IR spectrum of the crystal obtained is shown in Fig. 7.

A TG-DTA (thermogravimetry-differential thermal analysis) chart of the crystal obtained is shown in Fig. 11. The TG loss in weight as found by TG-DTA (not higher than 150°C) was 1.29%. In DTA, an exothermic peak temperature was found at 207.1°C (temperature programming: 5.0°C/minute).

[Example 4] Production of the δ crystal modification of the nonsolvate-form crystal of polymethine compound

The α crystal modification (10.00 g) obtained in Example 1 was added to 5 ml of methanol and 20 ml of acetone, and the mixture was heated to 60°C. After 30 minutes of stirring for dissolution at 60-65°C, 100 ml of ethyl acetate was added to the mixture, and the whole mixture was stirred at the same temperature for 1 hour. After cooling to 15-20°C, the crystalline precipitate was collected by filtration, washed with ethyl acetate and dried to give 9.07 g of the δ crystal modification of the compound of formula (I).

This crystal showed a solubility of not lower than 15% in each of methanol and ethanol. The elemental analysis data, melting point (decomposition temperature), absorption maximum wavelength (λmax) and gram extinction coefficient (εg) of this crystal were as follows.

| Elemental analysis ($C_{38}H_{41}ClN_2O_3S$): MW = 641.3 | | | |
|---|---|---|---|
| | C | H | N |
| Calculated (%) | 71.17 | 6.44 | 4.37 |
| Found (%) | 71.11 | 6.40 | 4.35 |

Melting point (°C): 198-199°C (decomposition)
λmax: 808 nm (diacetone alcohol solution)
εg: 4.43 x $10^5$ ml/g·cm

A powder X-ray diffraction pattern of the crystal obtained is shown in Fig. 4.

An IR spectrum of the crystal obtained is shown in Fig. 8.

A TG-DTA (thermogravimetry-differential thermal analysis) chart of the crystal obtained is shown in Fig. 12. The TG loss in weight as found by TG-DTA (not higher than 150°C) was 1.31%. In DTA, an exothermic peak temperature was found at 213.2°C (temperature programming: 5.0°C/minute).

[Example 5] Production of the δ crystal modification of the nonsolvate-form crystal of polymethine compound

The δ crystal modification of compound of formula (I) (8.40 g) was obtained by following the procedure of Example 4 in the same manner except that 9.0 g of the β crystal modification obtained in Example 2 was used in lieu of 10.0 g of the α crystal modification.

This crystal showed a solubility of not lower than 15% in each of methanol and ethanol. The elemental analysis data, melting point (decomposition temperature), absorption maximum wavelength (λmax) and gram extinction coefficient (εg) of this crystal were as follows.

Elemental analysis ($C_{38}H_{41}ClN_2O_3S$): MW = 641.3

| | C | H | N |
|---|---|---|---|
| Calculated (%) | 71.17 | 6.44 | 4.37 |
| Found (%) | 71.09 | 6.42 | 4.38 |

Melting point (°C): 198-199°C (decomposition)
λmax: 808 nm (diacetone alcohol solution)
εg: 4.42 x $10^5$ ml/g·cm

The crystal obtained gave the same powder X-ray diffraction pattern, IR spectrum and TG-DTA (thermogravimetry-differential thermal analysis) chart as those obtained in Example 4.

[Example 6] Production of the δ crystal modification of the nonsolvate-form crystal of polymethine compound

The δ crystal modification of compound of formula (I) (9.50 g) was obtained by following the procedure of Example 4 in the same manner except that 10.0 g of the γ crystal modification obtained in Example 3 was used in lieu of 10.0 g of the α crystal modification.

This crystal showed a solubility of not lower than 15% in each of methanol and ethanol. The elemental analysis data, melting point (decomposition temperature), absorption maximum wavelength (λmax) and gram extinction coefficient (εg) of this crystal were as follows.

Elemental analysis ($C_{38}H_{41}ClN_2O_3S$): MW = 641.3

| | C | H | N |
|---|---|---|---|
| Calculated (%) | 71.17 | 6.44 | 4.37 |
| Found (%) | 71.12 | 6.48 | 4.34 |

Melting point (°C): 198-199°C (decomposition)
λmax: 808 nm (diacetone alcohol solution)
εg: 4.41 x $10^5$ ml/g·cm

The crystal obtained gave the same powder X-ray diffraction pattern, IR spectrum and TG-DTA (thermogravimetry-differential thermal analysis) chart as those obtained in Example 4.

[Example 7] Production of the δ crystal modification of the nonsolvate-form crystal of polymethine compound

To 30 ml of acetone and 7.5 ml of methanol was added 15.03 g of a polymethine ether compound represented by the formula (II) (R = $CH_3$), and 6.00 g of p-toluenesulfonic acid monohydrate was added to the mixture with stirring at 25-30°C. The resulting mixture was stirred at that temperature for 1 hour and then heated to a temperature of 50-55°C, and 150 ml of ethyl acetate was added dropwise. After 1 hour of stirring at the same temperature, the mixture was cooled to 15-20°C. The resulting crystalline precipitate was collected by filtration, washed with ethyl acetate and then dried to

give 17.96 g of the δ crystal modification of the compound of formula (I).

This crystal showed a solubility of not lower than 15% in each of methanol and ethanol. The elemental analysis data, melting point (decomposition temperature), absorption maximum wavelength (λmax) and gram extinction coefficient (εg) of this crystal were as follows.

Elemental analysis ($C_{38}H_{41}ClN_2O_3S$): MW = 641.3

| | C | H | N |
|---|---|---|---|
| Calculated (%) | 71.17 | 6.44 | 4.37 |
| Found (%) | 71.12 | 6.41 | 4.38 |

Melting point (°C): 198-199°C (decomposition)
λmax: 808 nm (diacetone alcohol solution)
εg: 4.42 x $10^5$ ml/g·cm

The crystal obtained gave the same powder X-ray diffraction pattern, IR spectrum and TG-DTA (thermogravimetry-differential thermal analysis) chart as those obtained in Example 4.

[Example 8] Manufacture of a near-infrared absorbing material

A solution was prepared by dissolving 10 g of Delpet 80N (product of Asahi Chemical Industry; an acrylic resin) as a binder and 0.2 g of the α crystal modification obtained in Example 1 in 90 g of a mixed solvent composed of toluene/methyl ethyl ketone/methanol (1/1/0.1 by volume). This solution was applied, using a wire bar, to a polyethylene terephthalate (PET) film with an average thickness of 5 μm to a coat layer thickness, after drying, of about 5 μm. A near-infrared absorbing material specimen was thus obtained.

Laser beams emitted from a single-mode semiconductor laser (wavelength 830 nm) were condensed by means of a lens and directed at the surface of the specimen so that the beam diameter on that surface might amount to 10 μm. The semiconductor laser was adjusted so that the power of the laser beam arriving at the surface might be varied within the range of 50-200 mW. In this manner, the specimen was subjected to single pulse irradiation with a pulse width of 20 μs. After completion of irradiation, the specimen was observed under an optical microscope. It was confirmed that when the laser power arriving at the surface was 50 mW, a through hole with a diameter of about 10 μm was formed.

[Examples 9-11] Manufacture of near-infrared absorbing materials

Near-infrared absorbing material specimens were obtained in the same manner as in Example 8 except that the β crystal modification obtained in Example 2 (Example 9), the γ crystal modification obtained in Example 3 (Example 10) or the δ crystal modification obtained in Example 7 (Example 11) was used in lieu of the α crystal modification.

The specimens were subjected to the same laser beam irradiation test as in Example 8 and, for all the three specimens, it was confirmed that when the laser power arriving at the surface was 50 mW, a through hole with a diameter of about 10 μm was formed.

[Comparative Example 1] Polymethine compound synthesis (cf. Japanese Kokai Publication 2002-52855, Synthesis Example 1)

Acetic anhydride (40 g) was added to a mixture composed of 8.74 g of an indoline compound represented by the formula (IV) ($R_1$=$CH_3$), 4.80g of a diformyl compound represented by the formula (V)(n = 1), 4.80 g of p-toluenesulfonic acid and 2.00 g of anhydrous sodium acetate, the mixture was heated to the refluxing temperature and stirred at that temperature for 1,0 hour. After cooling to room temperature, the reaction mixture was subjected to suction filtration to remove insoluble impurities. This reaction mixture was then poured into 120 g of ice-water, and the solid precipitate was collected by filtration, washed with methanol and dried at 60°C. Thus was obtained 0.85 g of a compound identical in chemical structure to the compound of the present invention.

The melting point, absorption maximum wavelength (λmax) and gram extinction coefficient (εg) of the compound obtained were as follows.

Melting point (°C): 145-160°C (indefinite)
λmax: 808 nm (diacetone alcohol solution)
εg: 3.52 x $10^5$ ml/g·cm

A powder X-ray diffraction pattern of the compound obtained is shown in Fig. 13.

A TG-DTA (thermogravimetry-differential thermal analysis) chart of the compound obtained is shown in Fig. 14. The TG loss in weight as found by TG-DTA (not higher than 150°C) was about 3.7%. In DTA, two exothermic peak

temperatures were found at 170.9°C and 193.4°C (temperature programming: 5.0°C/minute).

The water content of this compound was determined on a Karl Fischer water-content meter. The water content was 3.6%. It was thus revealed that the TG weight loss is due to the water content and this compound is a hydrate.

<Stability in solution>

A solution stability test was carried out in the following manner. The results are shown in Table 1.

Each polymethine compound specified below in Table 1 was dissolved in a mixture of ethanol and methyl ethyl ketone (1/1 by volume) to a concentration of 5% (w/v) and the solution was allowed to stand in a room (at room temperature) for 10 days. The absorbance (gram extinction coefficient) of the solution was measured before and after standing, and the decomposition percentage was calculated using the following formula.

Decomposition percentage (%) = [(absorbance just after preparation of solution – absorbance after 10 days of standing)/(absorbance just after preparation of solution)] x 100

[Table 1]

| polymethine compound | gram extinction coeffcient (εg) | TG weight loss (up to 150°C) | Decomposition percentage |
|---|---|---|---|
| Example 1 α crystal modification | $4.42\times10^5$ | 0.06% | 2.0% |
| Example 2 β crystal modification | $4.41\times10^5$ | 1.23% | 2.1% |
| Example 3 γ crystal modification | $4.41\times10^5$ | 1.29% | 2.2% |
| Example 4 δ crystal modification | $4.43\times10^5$ | 1.31% | 1.9% |
| Comparative Example 1 (hydrate) | $3.52\times10^5$ | 3.7 % | 8.1% |

In the case of preparing original plates for plate making or photograving, for instance, when the polymethine compound solution is poor in stability, the stock solution cannot be prepared or stored in large amounts, hence the production efficiency declines. Further, the decomposition of the polymethine compound, even if in a small percentage, may lead to changes in light-to-heat conversion efficiency or changes in color tone in original plates for plate making, which is unfavorable from the product quality viewpoint.

Industrial Applicability

The nonsolvate-form crystal of polymethine compound of the invention is highly stable in solution and therefore is easy to handle. It has a high gram extinction coefficient and therefore is highly sensitive to general-purpose semiconductor lasers. Further, it is highly soluble in alcohol solvents. Thus, it is very useful in the fields of recording materials and plate making materials where laser beams are utilized.

Brief Description of the Drawinqs

[Fig. 1] This is a powder X-ray diffraction pattern of the α crystal modification of nonsolvate-form crystal of polymethine compound of Example 1.

[Fig. 2] This is a powder X-ray diffraction pattern of the β crystal modification of nonsolvate-form crystal of polymethine compound of Example 2.

[Fig. 3] This is a powder X-ray diffraction pattern of the γ crystal modification of nonsolvate-form crystal of polymethine

compound of Example 3.

[Fig. 4] This is a powder X-ray diffraction pattern of the δ crystal modification of nonsolvate-form crystal of polymethine compound of Example 4.

[Fig. 5] This is an IR absorption spectrum of the α crystal modification of nonsolvate-form crystal of polymethine compound of Example 1.

[Fig. 6] This is an IR absorption spectrum of the β crystal modification of nonsolvate-form crystal of polymethine compound of Example 2.

[Fig. 7] This is an IR absorption spectrum of the γ crystal modification of nonsolvate-form crystal of polymethine compound of Example 3.

[Fig. 8] This is an IR absorption spectrum of the δ crystal modification of nonsolvate-form crystal of polymethine compound of Example 4.

[Fig. 9] This is a TG-DTA (thermogravimetry-differential thermal analysis) chart of the α crystal modification of nonsolvate-form crystal of polymethine compound of Example 1.

[Fig. 10]This is a TG-DTA (thermogravimetry-differential thermal analysis) chart of the β crystal modification of nonsolvate-form crystal of polymethine compound of Example 2.

[Fig. 11]This is a TG-DTA (thermogravimetry-differential thermal analysis) chart of the γ crystal modification of nonsolvate-form crystal of polymethine compound of Example 3.

[Fig. 12]This is a TG-DTA (thermogravimetry-differential thermal analysis) chart of the δ crystal modification of nonsolvate-form crystal of polymethine compound of Example 4.

[Fig. 13]This is a powder X-ray diffraction pattern of the compound of Comparative Example 1.

[Fig. 14]This is a TG-DTA (thermogravimetry-differential thermal analysis) chart of the compound of Comparative Example 1.

## Claims

**1.** A nonsolvate-form crystal of a polymethine compound represented by the formula (I):

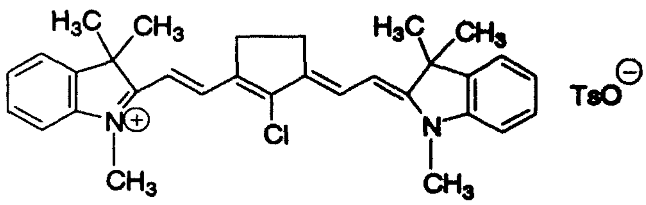

(I)

wherein TsO represents the p-toluenesulfonic acid residue.

**2.** The nonsolvate-form crystal of polymethine compound according to Claim 1 which shows a thermogravimetric (TG) weight loss not exceeding 3% at up to 150°C in a TG-TDA (thermogravimetry-differential thermal analysis) chart thereof.

**3.** A process for producing the nonsolvate-form crystal of polymethine compound according to Claim 1 or 2 which comprises reacting a polymethine ether compound represented by the formula (II) with p-toluenesulfonic acid.

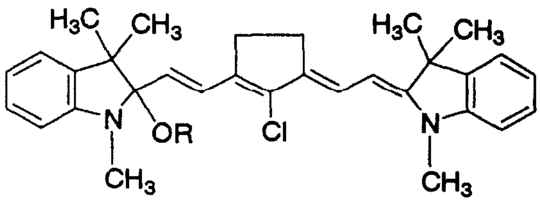

(II)

(In the formula, R represents an alkyl or alkoxyalkyl group or an aryl group which may optionally be substituted.)

4. The α crystal modification of the nonsolvate-form crystal of polymethine compound according to Claim 1 or 2 which is **characterized by** a powder X-ray diffraction pattern thereof showing characteristic peaks at diffraction angles (2θ ± 0.2°) of 11.7°, 16.3°, 17.0°, 24.8° and 27.5° in powder X-ray diffractometry using the Cu-Kα rays.

5. A process for producing the α crystal modification of the nonsolvate-form crystal of polymethine compound according to Claim 4 which comprises using a polymethine ether compound represented by the formula (II) and p-toluenesulfonic acid as reactants and obtaining the crystal in the presence of a mixed solvent composed of a ketone solvent and an ester solvent.

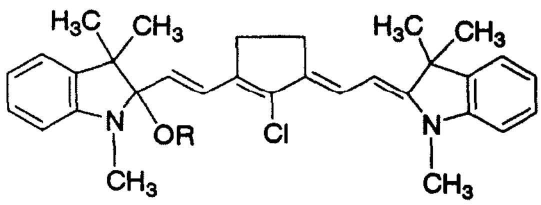

(II)

(In the formula, R represents an alkyl or alkoxyalkyl group or an aryl group which may optionally be substituted.)

6. The β crystal modification of the nonsolvate-form crystal of polymethine compound according to Claim 1 or 2 which is **characterized by** a powder X-ray diffraction pattern thereof showing characteristic peaks at diffraction angles (2θ ± 0.2°) of 15.5°, 19.0°, 22.3° and 22.9° in powder X-ray diffractometry using the Cu-Kα rays.

7. A process for producing the β crystal modification of the nonsolvate-form crystal of polymethine compound according to Claim 6 which comprises subjecting the α crystal modification according to Claim 4 to dispersion treatment in warm water at a temperature not lower than 30°C.

8. The γ crystal modification of the nonsolvate-form crystal of polymethine compound according to Claim 1 or 2 which is **characterized by** a powder X-ray diffraction pattern thereof showing characteristic peaks at diffraction angles (2θ ± 0.2°) of 11.6°, 11.9° and 24.5° in powder X-ray diffractometry using the Cu-Kα rays.

9. A process for producing the γ crystal modification according to Claim 8 which comprises using a polymethine ether compound represented by the formula (II) and p-toluenesulfonic acid as reactants and causing the crystal to precipitate out from a mixed solvent composed of an alcohol solvent and water.

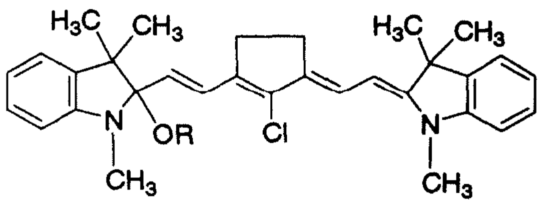

(II)

(In the formula, R represents an alkyl or alkoxyalkyl group or an aryl group which may optionally be substituted.)

10. The δ crystal modification of the nonsolvate-form crystal of polymethine compound according to Claim 1 or 2 which is **characterized by** a powder X-ray diffraction pattern thereof showing characteristic peaks at diffraction angles (2θ ± 0.2°) of 10.8°, 14.8°, 16.9°, 21.4° and 23.7° in powder X-ray diffractometry using the Cu-Kα rays.

11. A process for producing the δ crystal modification according to Claim 10 which comprises dissolving at least one of the α, β and γ crystal modifications defined in Claims 4, 6 and 8, respectively, in a mixed solvent composed of a ketone solvent and an alcohol solvent and causing the crystal to precipitate out therefrom by addition of an ester solvent.

**12.** A process for producing the δ crystal modification according to Claim 10 which comprises using a polymethine ether compound represented by the formula (II) and p-toluenesulfonic acid as reactants, carrying out the reaction using a mixed solvent composed of a ketone solvent and an alcohol solvent and then adding an ester solvent to the reaction mixture for causing the crystal to precipitate out.

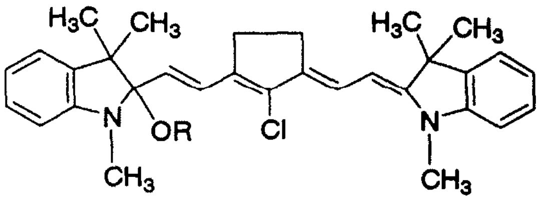

(II)

(In the formula, R represents an alkyl or alkoxyalkyl group or an aryl group which may optionally be substituted.)

**13.** A near-infrared absorbing material **characterized by** containing the nonsolvate-form crystal of polymethine compound according to Claim 1, 2, 4, 6, 8 or 10.

FIG. 1

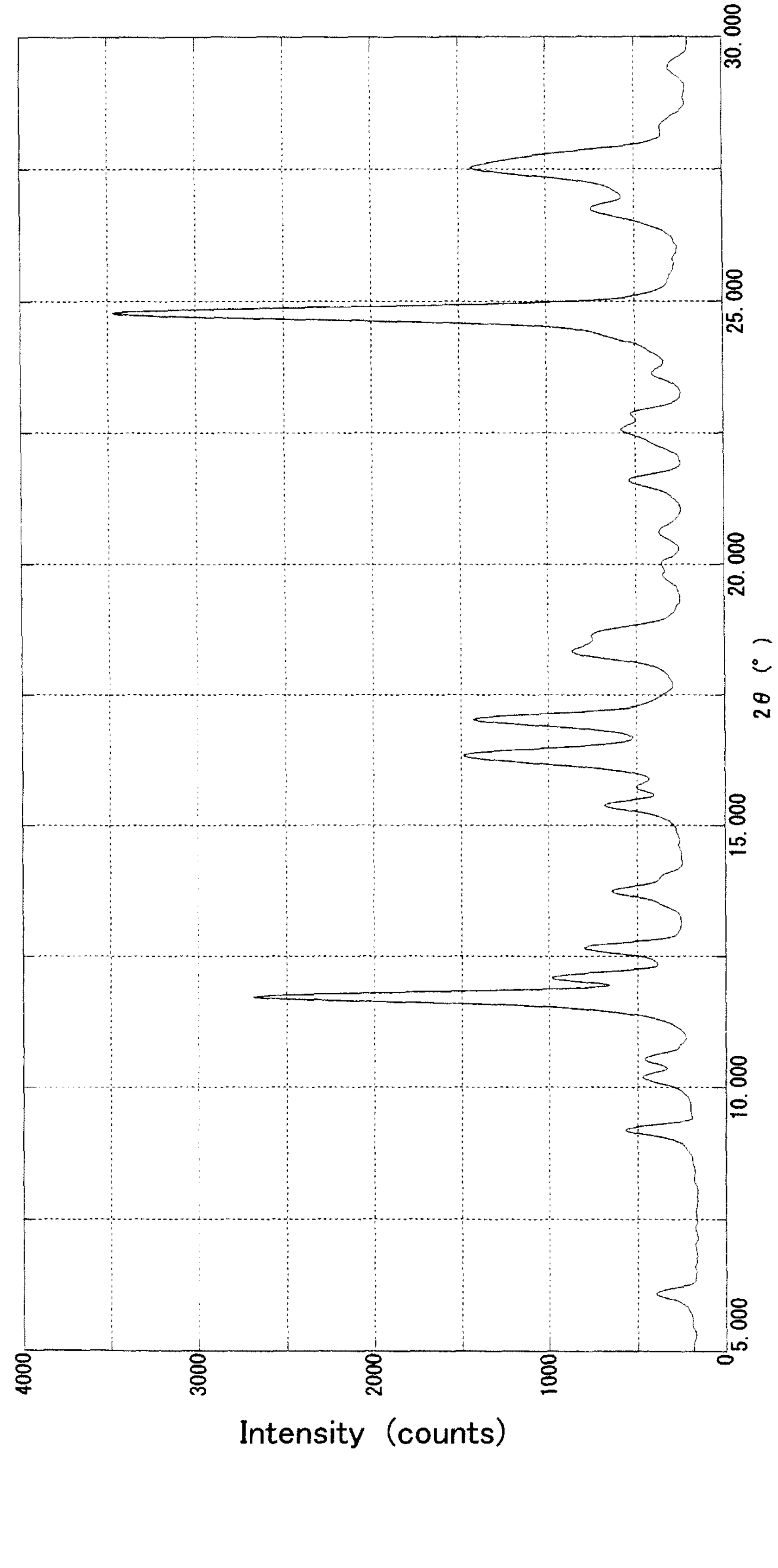
4000
3000
2000
1000
0
Intensity (counts)
5.000
10.000
15.000
20.000
25.000
30.000
2θ (°)

FIG. 2

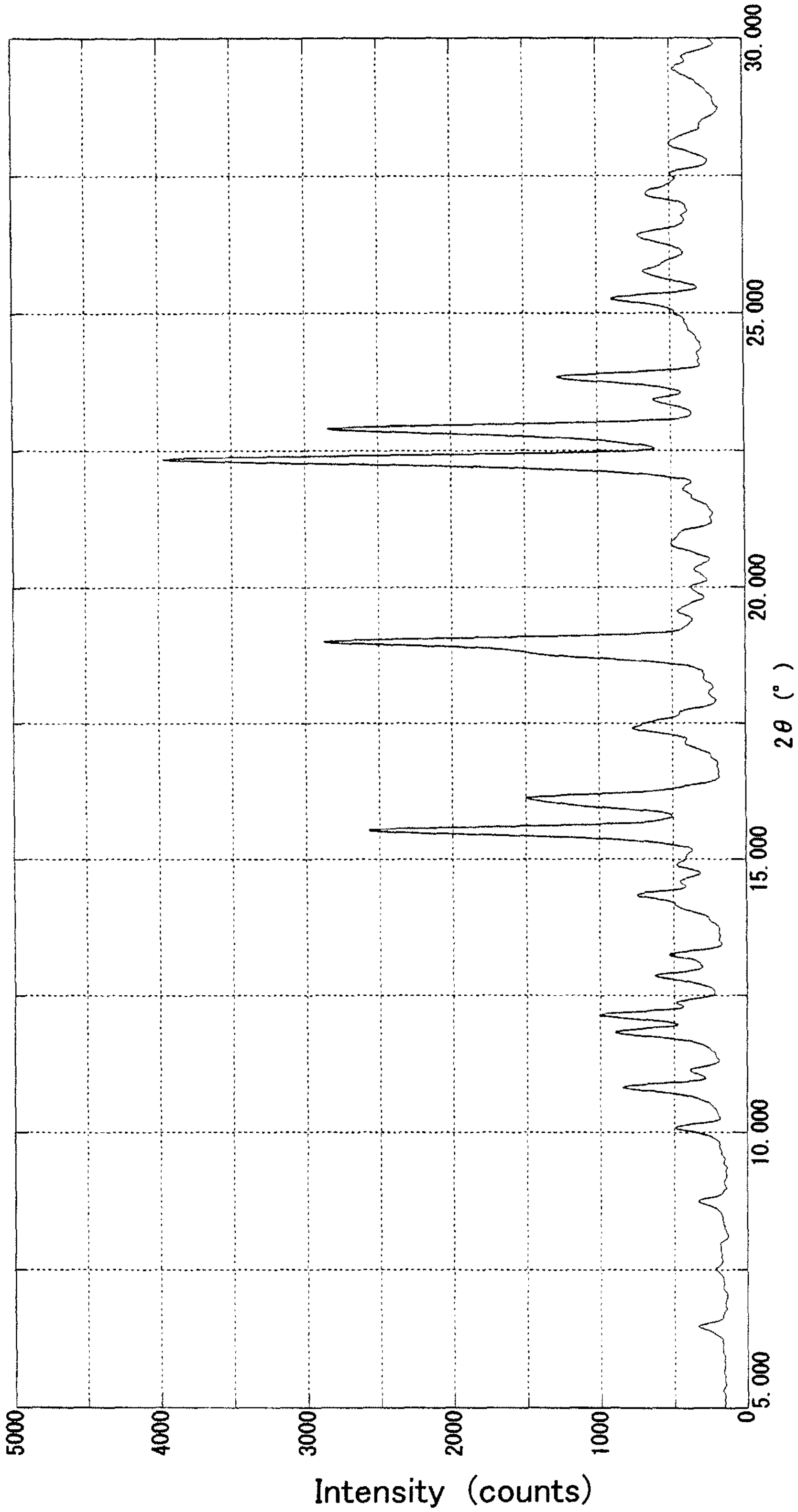
Intensity (counts)
2θ (°)
0
1000
2000
3000
4000
5000
5.000
10.000
15.000
20.000
25.000
30.000

FIG. 3

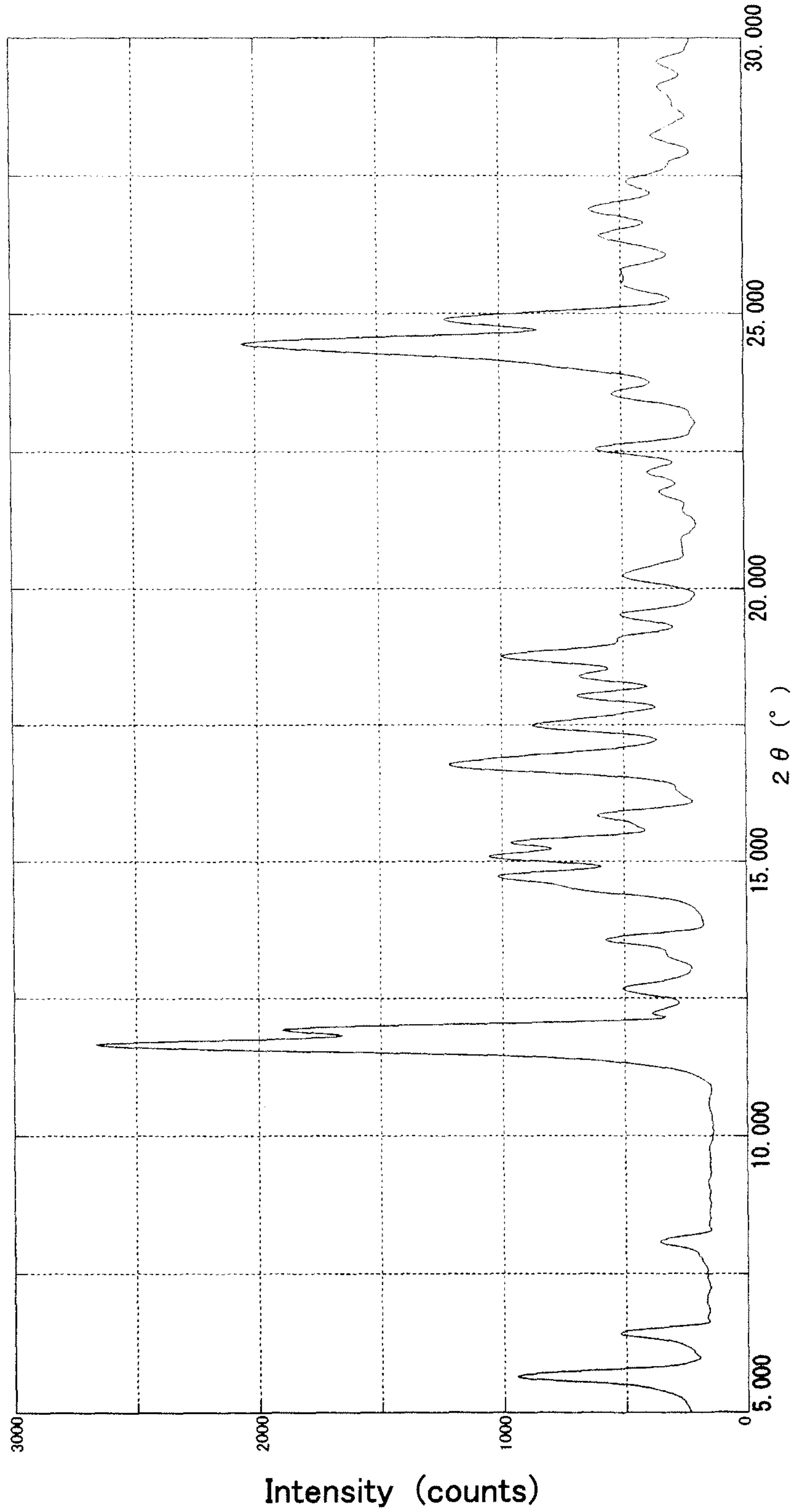
5.000
10.000
15.000
20.000
25.000
30.000
2θ (°)
0
1000
2000
3000
Intensity (counts)

FIG. 4

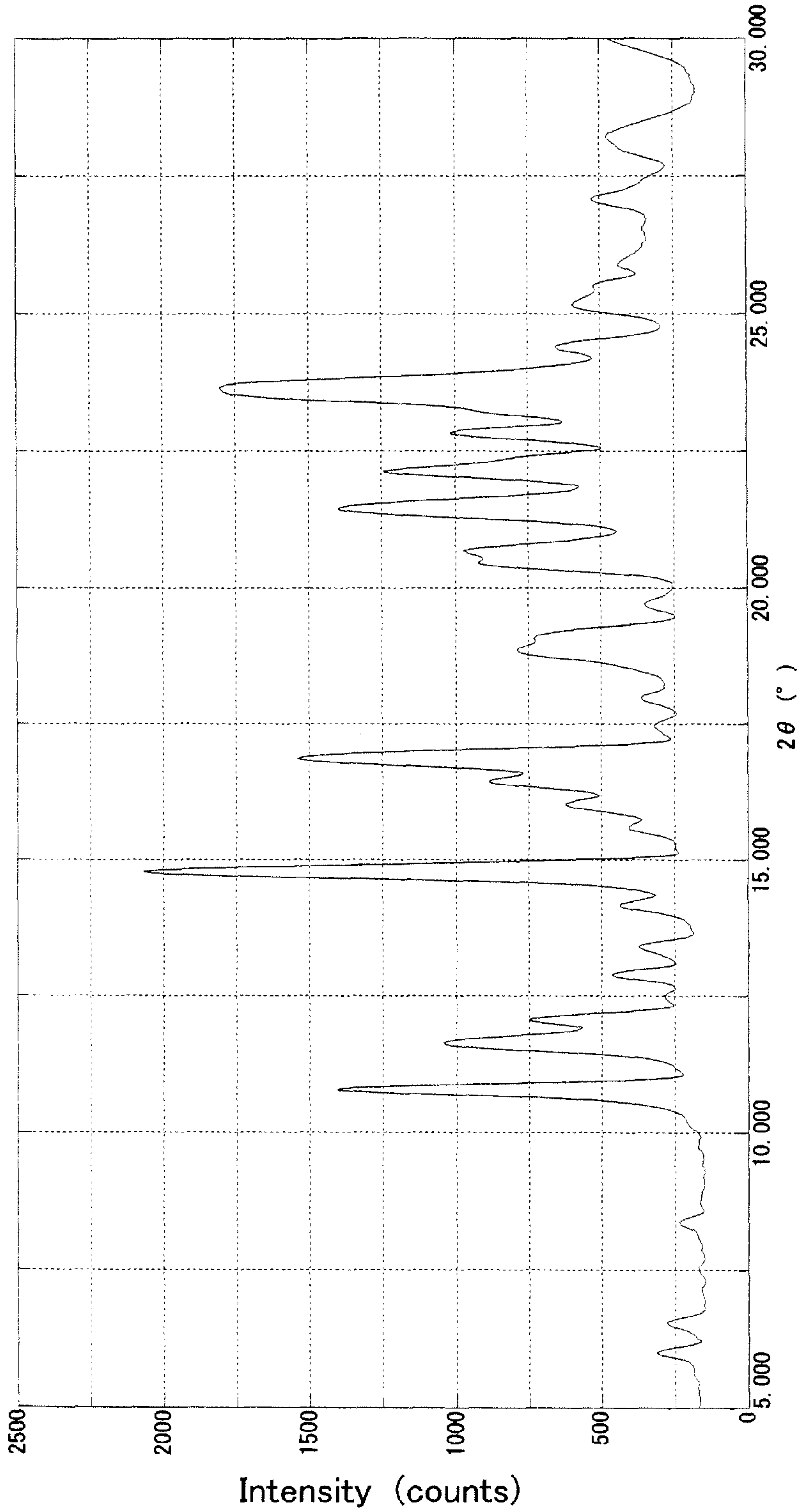
2θ (°)
5.000
10.000
15.000
20.000
25.000
30.000
Intensity (counts)
0
500
1000
1500
2000
2500

FIG. 5

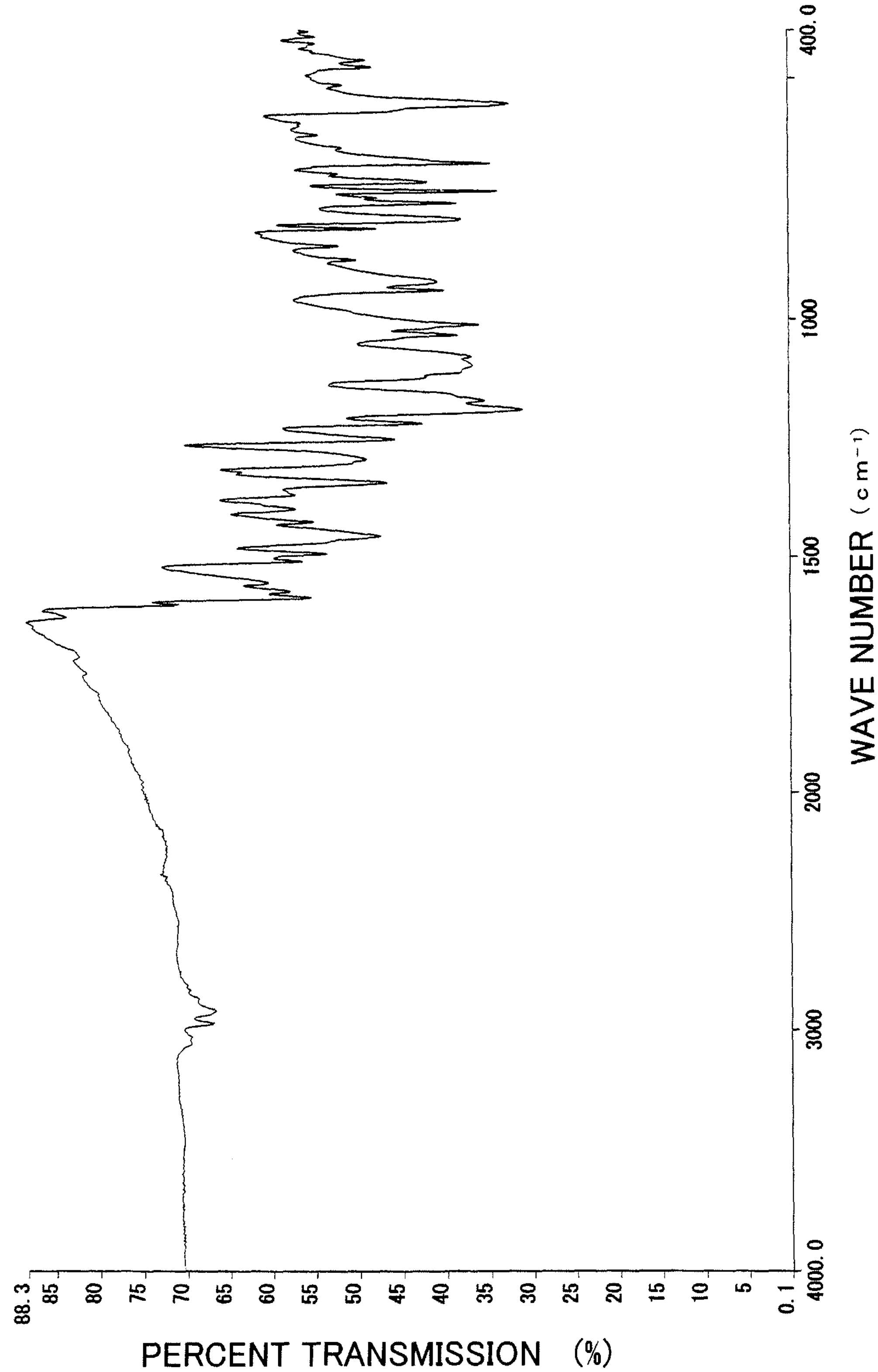
PERCENT TRANSMISSION (%)
88.3
85
80
75
70
65
60
55
50
45
40
35
30
25
20
15
10
5
0.1
WAVE NUMBER (cm$^{-1}$)
4000.0
3000
2000
1500
1000
400.0

FIG. 6

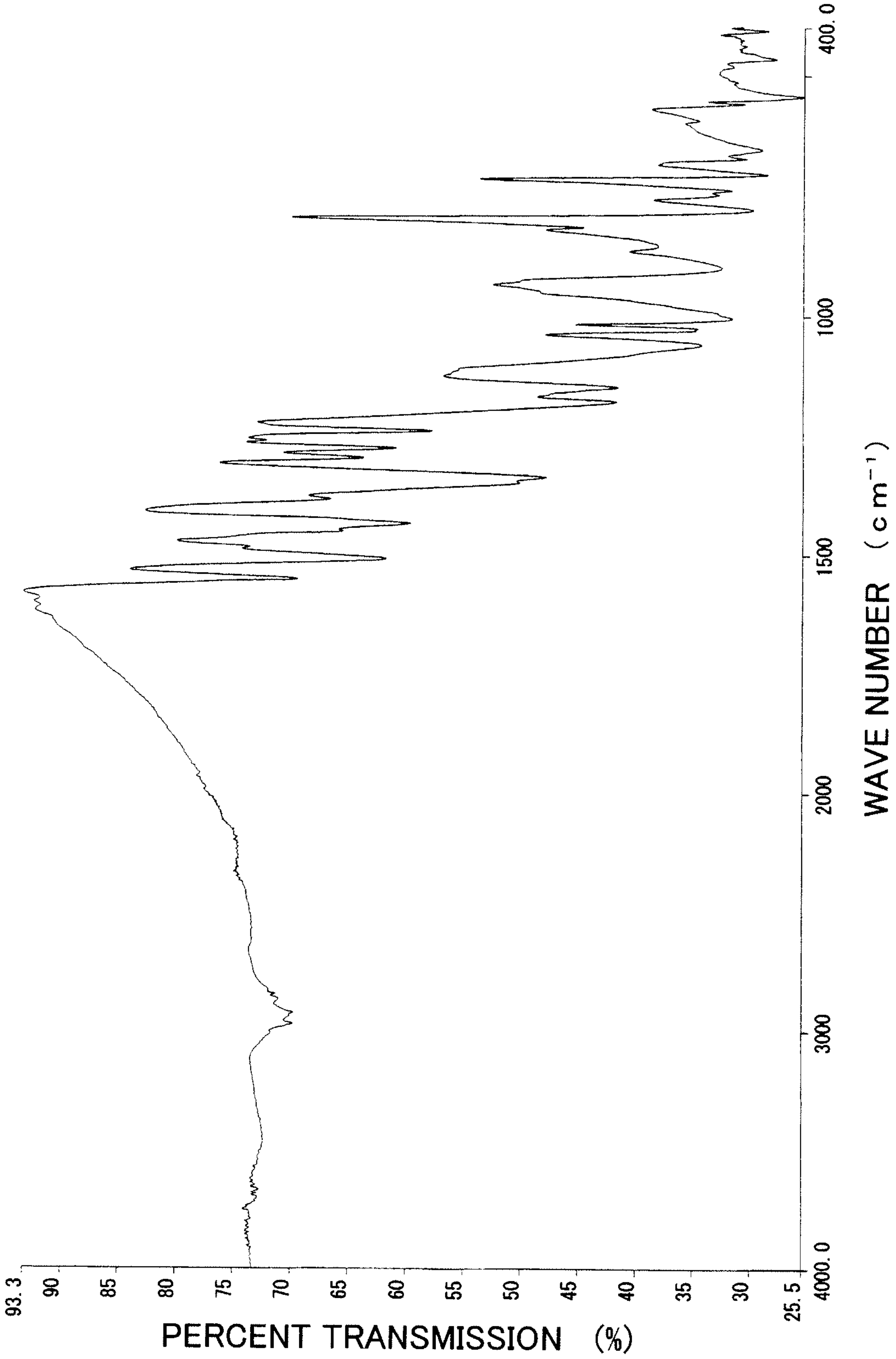
PERCENT TRANSMISSION (%)
93.3
90
85
80
75
70
65
60
55
50
45
40
35
30
25.5
WAVE NUMBER (cm$^{-1}$)
4000.0
3000
2000
1500
1000
400.0

FIG. 7

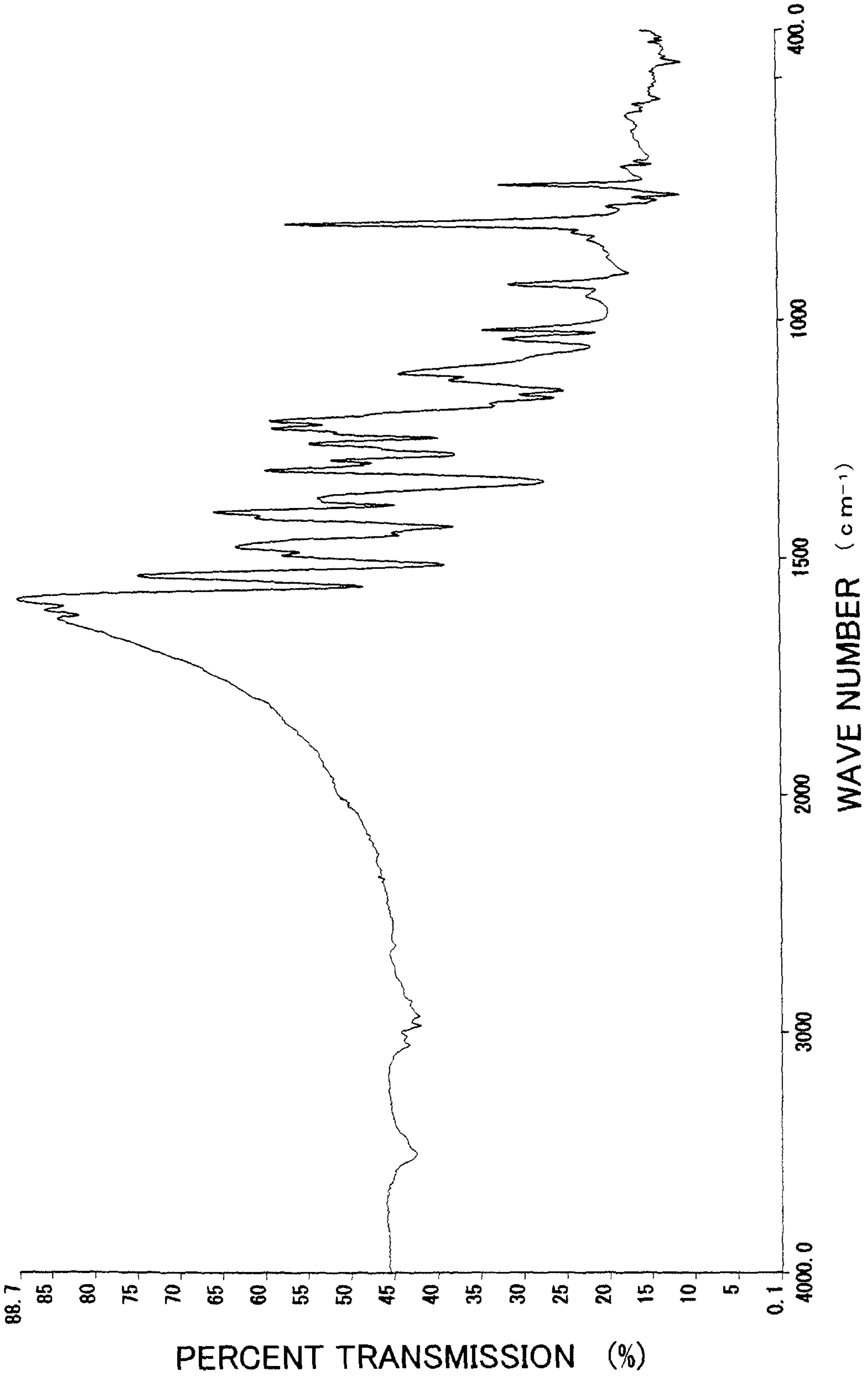
WAVE NUMBER (cm⁻¹)
4000.0
3000
2000
1500
1000
400.0
PERCENT TRANSMISSION (%)
88.7
85
80
75
70
65
60
55
50
45
40
35
30
25
20
15
10
5
0.1

FIG. 8

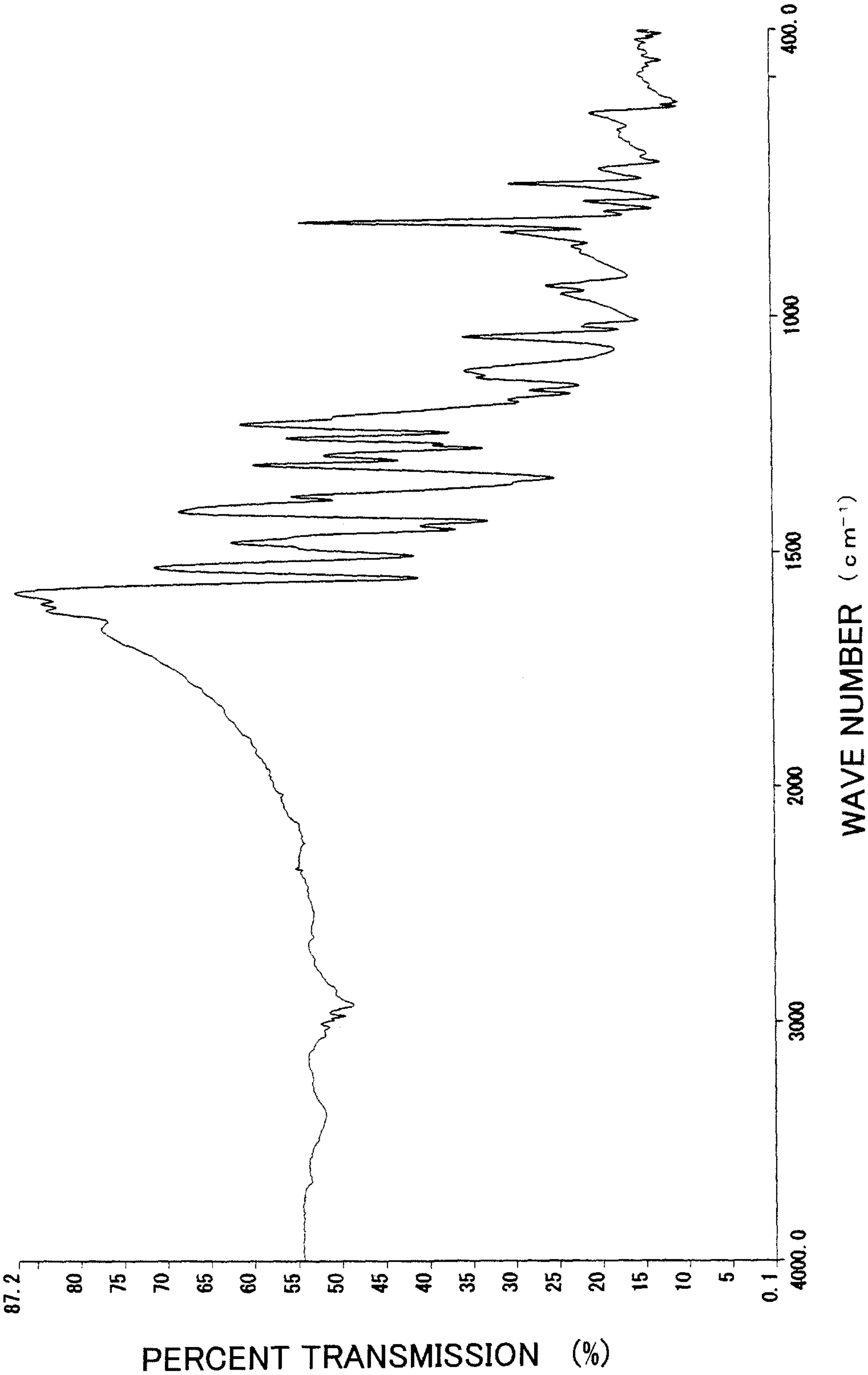
PERCENT TRANSMISSION (%)
87.2
80
75
70
65
60
55
50
45
40
35
30
25
20
15
10
5
0.1
WAVE NUMBER (cm⁻¹)
4000.0
3000
2000
1500
1000
400.0

FIG. 9

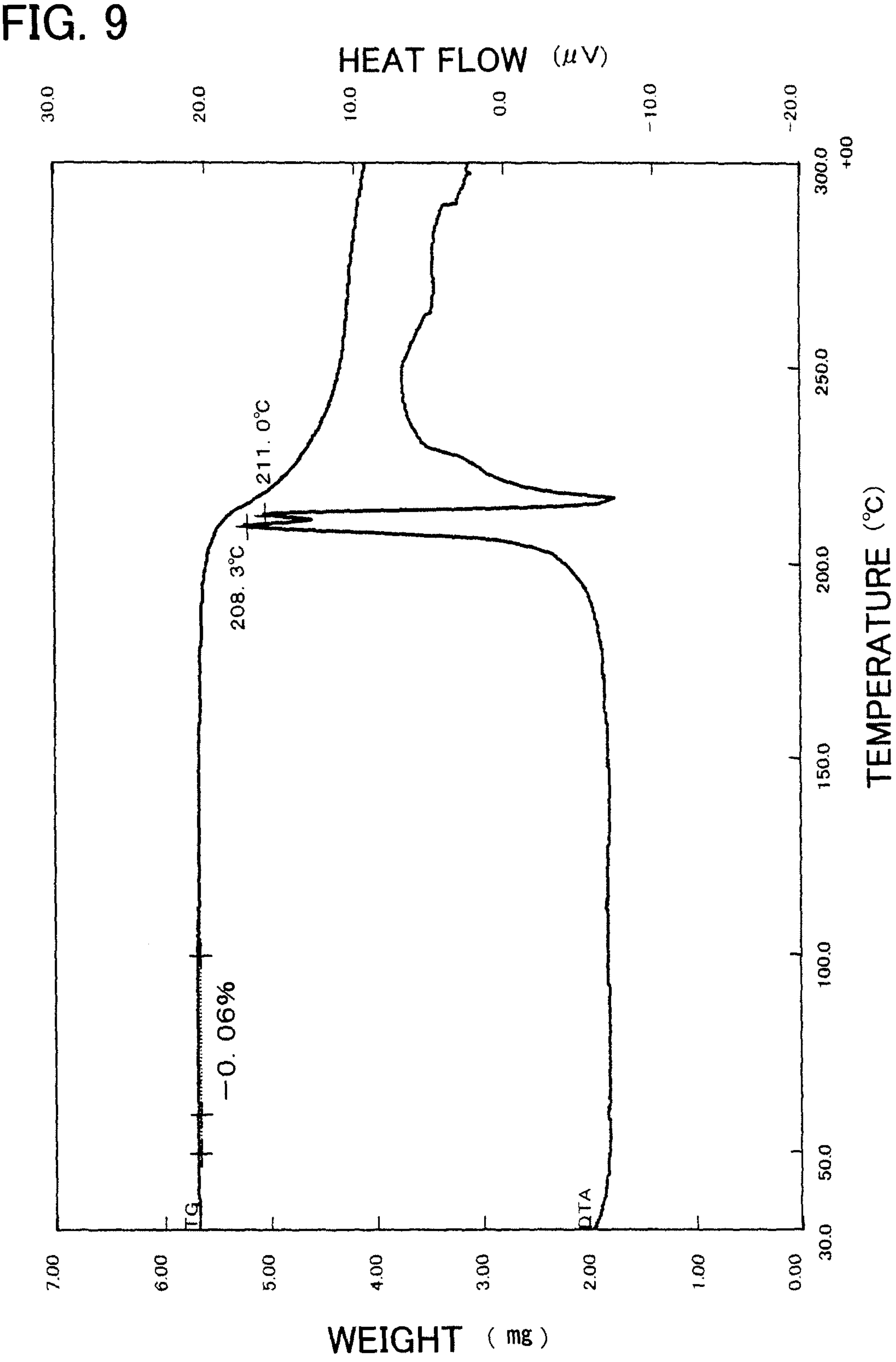
HEAT FLOW (μV)
30.0
20.0
10.0
0.0
−10.0
−20.0
+00
300.0
250.0
200.0
150.0
100.0
50.0
30.0
TEMPERATURE (°C)
211. 0°C
208. 3°C
−0. 06%
TG
DTA
7.00
6.00
5.00
4.00
3.00
2.00
1.00
0.00
WEIGHT (mg)

FIG. 10

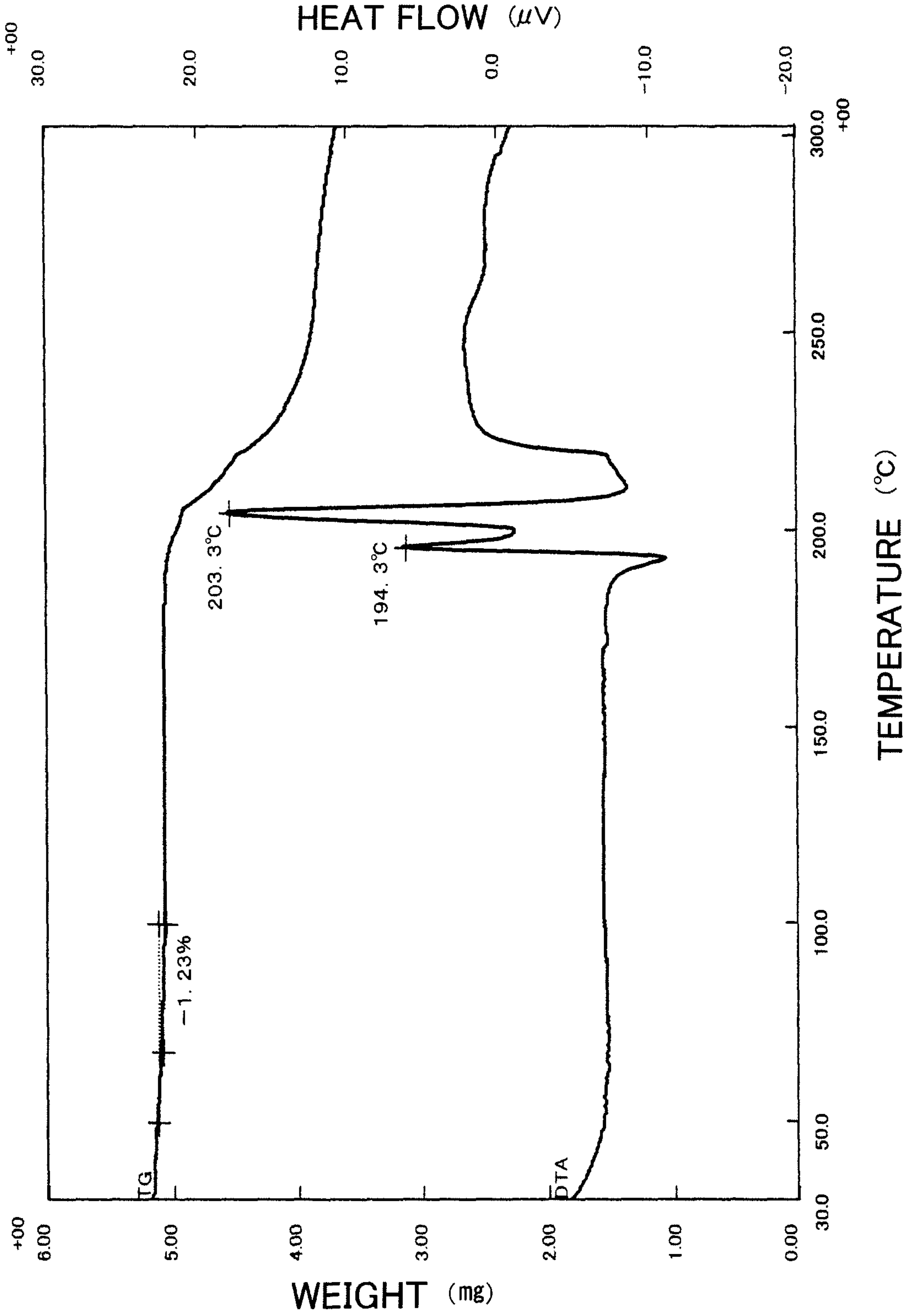
HEAT FLOW (μV)
+00
30.0
20.0
10.0
0.0
−10.0
−20.0
+00
300.0
250.0
200.0
150.0
100.0
50.0
30.0
TEMPERATURE (°C)
203. 3°C
194. 3°C
−1. 23%
TG
DTA
+00
6.00
5.00
4.00
3.00
2.00
1.00
0.00
WEIGHT (mg)

FIG. 11

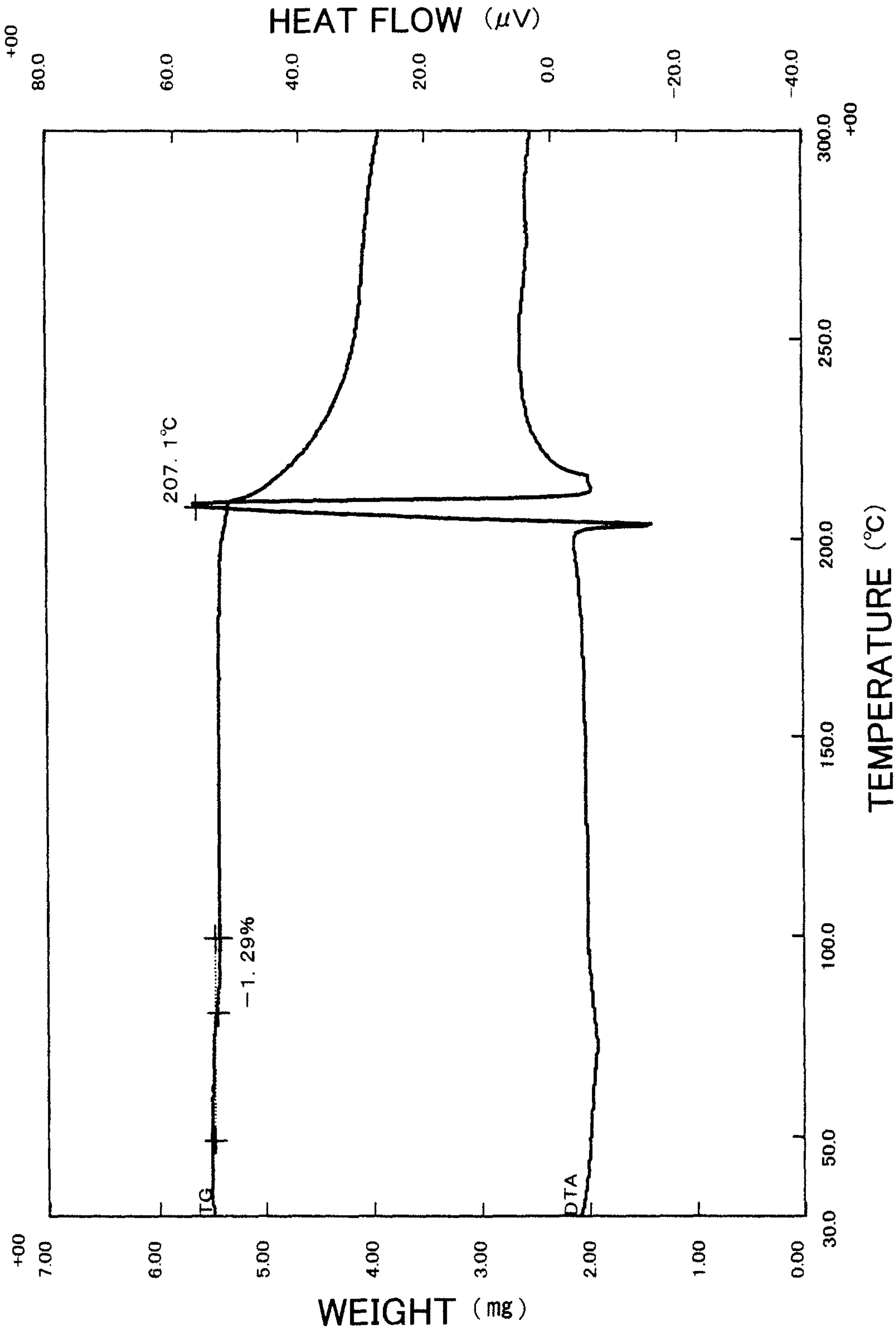
HEAT FLOW (μV)
80.0
60.0
40.0
20.0
0.0
-20.0
-40.0
+00
207. 1°C
-1. 29%
TG
DTA
WEIGHT (mg)
7.00
6.00
5.00
4.00
3.00
2.00
1.00
0.00
TEMPERATURE (°C)
30.0
50.0
100.0
150.0
200.0
250.0
300.0

FIG. 12

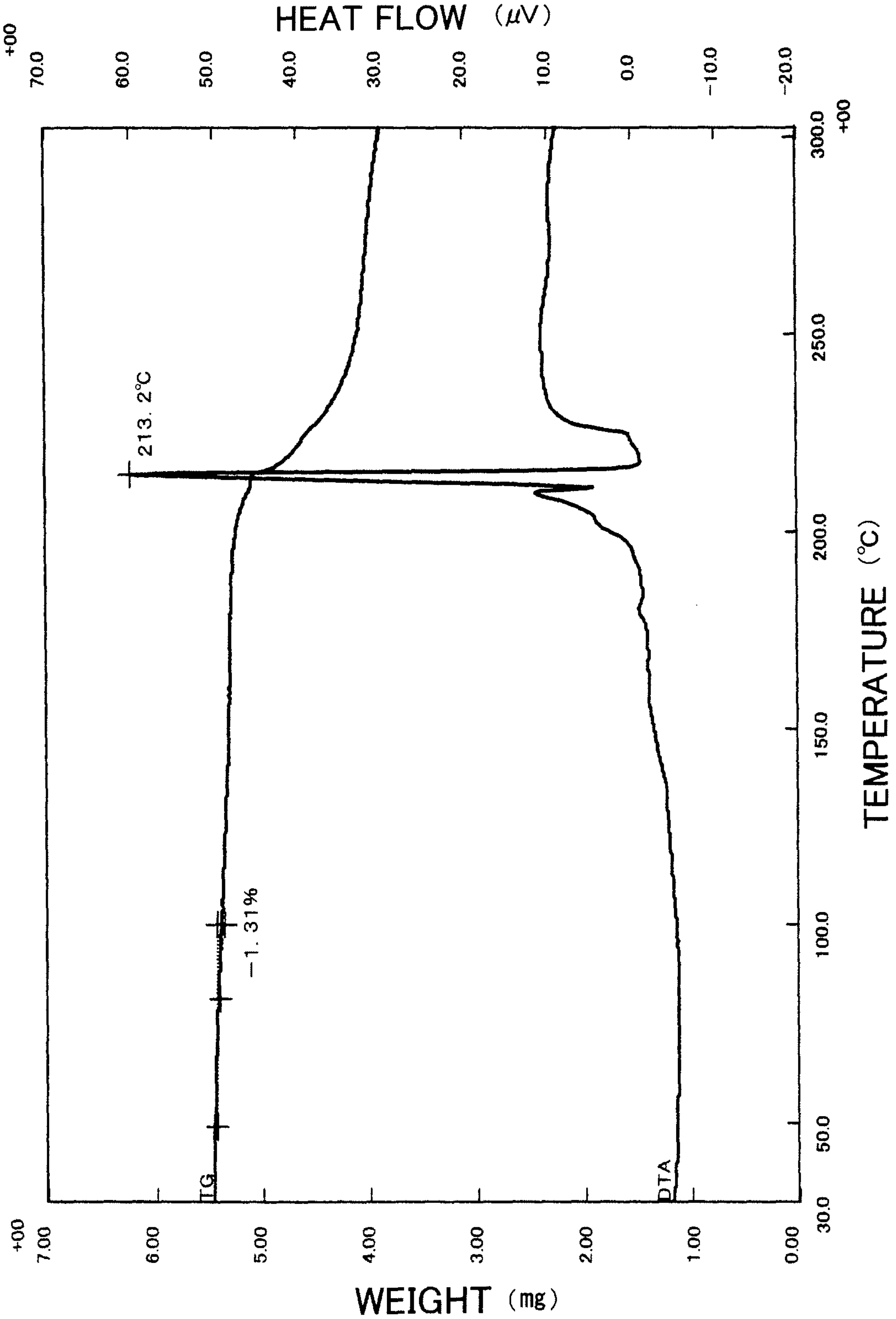
HEAT FLOW (μV)
+00
70.0
60.0
50.0
40.0
30.0
20.0
10.0
0.0
-10.0
-20.0
213. 2°C
-1. 31%
TG
DTA
+00
300.0
250.0
200.0
150.0
100.0
50.0
30.0
TEMPERATURE (°C)
+00
7.00
6.00
5.00
4.00
3.00
2.00
1.00
0.00
WEIGHT (mg)

FIG. 13

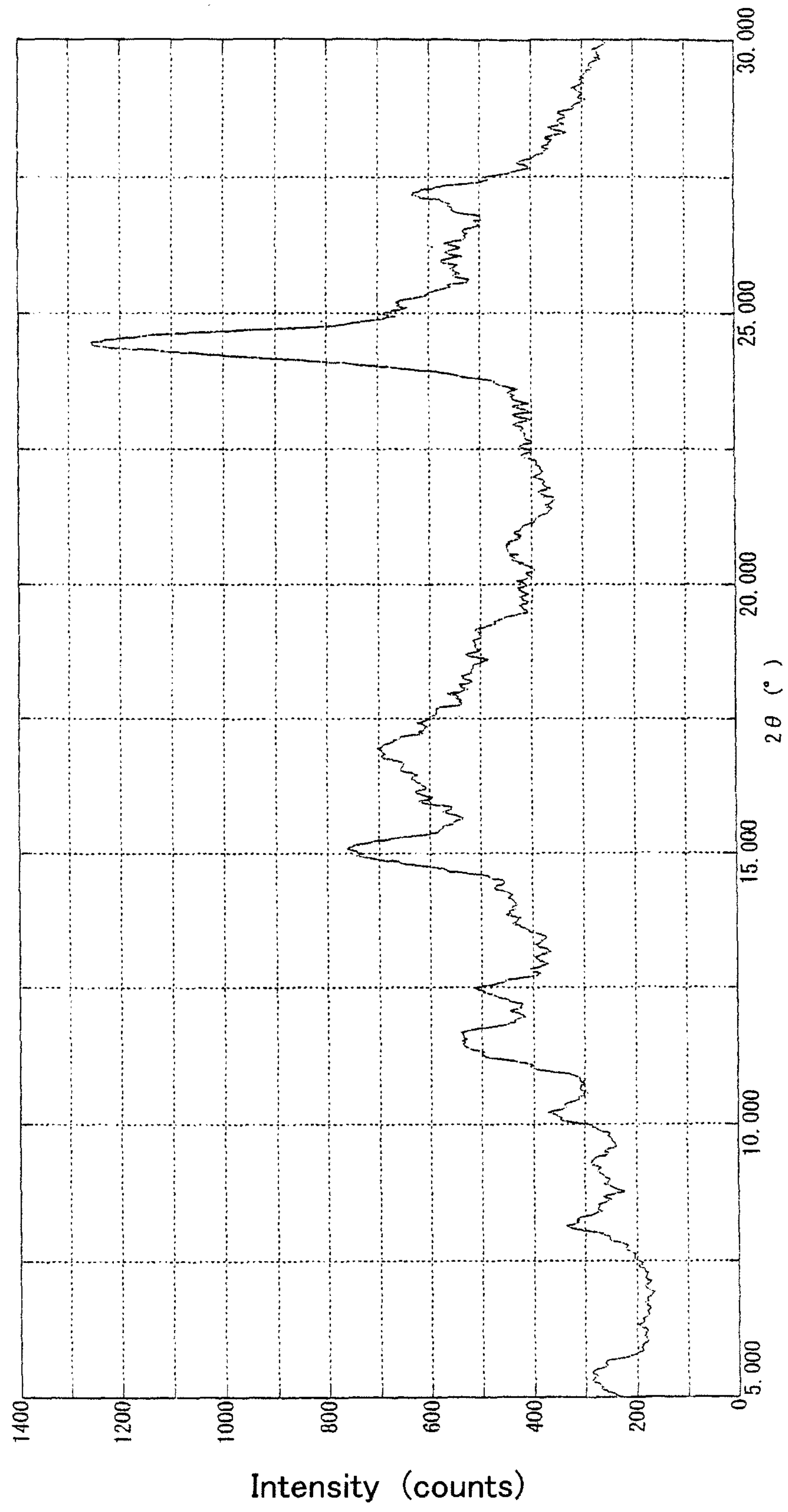
30.000
25.000
20.000
15.000
10.000
5.000
2θ (°)
1400
1200
1000
800
600
400
200
0
Intensity (counts)

FIG. 14

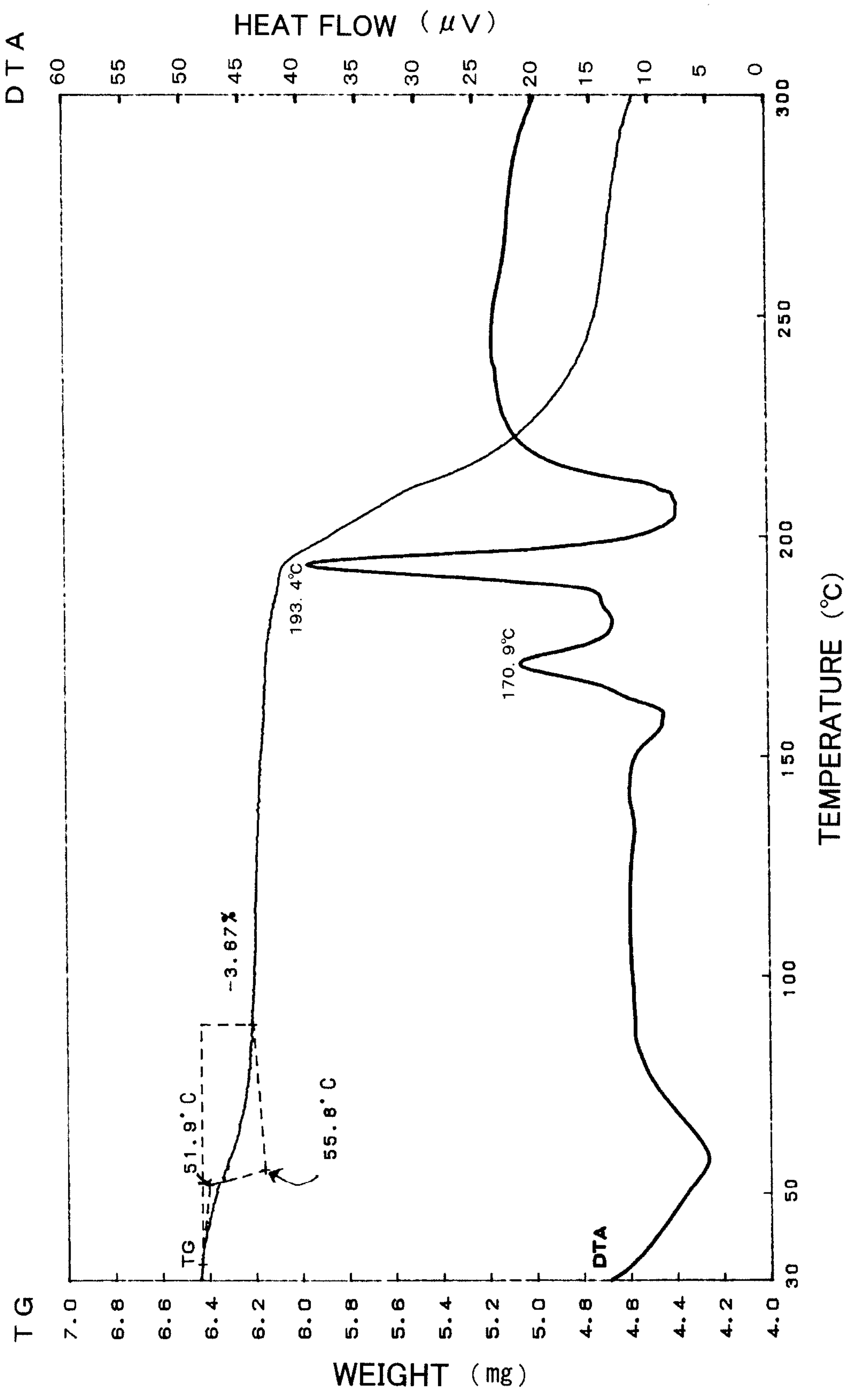
DTA
HEAT FLOW (μV)
60 55 50 45 40 35 30 25 20 15 10 5 0
TG
7.0 6.8 6.6 6.4 6.2 6.0 5.8 5.6 5.4 5.2 5.0 4.8 4.6 4.4 4.2 4.0
WEIGHT (mg)
30 50 100 150 200 250 300
TEMPERATURE (°C)
193.4°C
170.9°C
-3.67%
51.9°C
55.8°C
TG
DTA

**INTERNATIONAL SEARCH REPORT**

International application No.
PCT/JP2005/022653

A. CLASSIFICATION OF SUBJECT MATTER
*C09B23/00*(2006.01), *C07D209/10*(2006.01), *C09B67/48*(2006.01), *C09K3/00*
(2006.01)

According to International Patent Classification (IPC) or to both national classification and IPC

B. FIELDS SEARCHED

Minimum documentation searched (classification system followed by classification symbols)
C07D209/10, C09B23/00, C09B67/48, C09K3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2004-219650 A (Fuji Photo Film Co., Ltd.), 05 August, 2004 (05.08.04), & US 2004/157152 A1 | 1-13 |
| A | JP 2001-133966 A (Fuji Photo Film Co., Ltd.), 18 May, 2001 (18.05.01), (Family: none) | 1-13 |
| P,A | WO 2001/113453 A1 (Yamamoto Chemicals, Inc.), 29 December, 2004 (29.12.04), (Family: none) | 1-13 |
| P,A | WO 2005/000814 A1 (Yamamoto Chemicals, Inc.), 06 January, 2005 (06.01.05), (Family: none) | 1-13 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February, 2006 (01.02.06) | 14 February, 2006 (14.02.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001064255 A
- JP 2002052855 A
- JP H10195319 A
- JP 3045404 B
- DE 3721850
- JP 62036469 A
- JP 2000035669 A
- JP 2000226528 A


### Non-patent literature cited in the description


- *Zh. Org. Khim.,* 1978, vol. 14 (10
- *J. Org. Chem.,* 1995, vol. 60, 2394
- Near-Infrared Absorbing Colors. Kagaku Kogyo. May 1986, 45-51
- Development and Market Trend of Functional Colors in the Nineties. CMC, 1990